# EUROPEAN PATENT SPECIFICATION

(11) **EP 2 983 657 B1**
(45) Date of publication and mention of the grant of the patent: **03.06.2020**
(21) Application number: 14778534.9
(22) Date of filing: 04.04.2014
(51) Int. Cl.: A61K 31/135, A61K 31/138, A61K 31/5355, A61K 31/5377, A61K 31/573, A61K 45/06, A61K 9/00, A61K 9/06, A61K 9/48, A61P 1/00, A61P 1/10, A61P 1/12, A61P 43/00

(54) **NADOLOL FORMULATIONS FOR USE IN THE TREATMENT OF INFLAMMATORY BOWEL DISORDERS**
NADOLOLFORMULIERUNGEN ZUR BEHANDLUNG VON CHRONISH-ENTZÜNDLICHEN DARMERKRANKUNGEN
PRÉPARATIONS DE NADOLOL DESTINÉES AU TRAITEMENT DES MALADIES INFLAMMATOIRES CHRONIQUES DE L'INTESTIN

(30) Priority: 05.04.2013 US 201361809148 P
(43) Date of publication of application: 17.02.2016
(73) Proprietor: Numedii, Inc., Palo Alto, CA 94303 (US)
(72) Inventor: DUDLEY, Joel, Rye, NY 10580 (US); CHRISTOPHER, Carol, Palo Alto, CA 94303 (US); PASRICHA, Pankaj, Columbia, MD 21044 (US)
(74) Representative: Avidity IP
(86) International application number: PCT/US2014/033080
(87) International publication number: WO 2014/165823

(56) References cited:
- WO-A1-93/00081
- US-A1- 2005 143 378
- US-A1- 2008 025 966
- J.F. FIELDING: "Timolol Treatment in the Irritable Bowel Syndrome", DIGESTION, vol. 22, no. 3, 1 January 1981 (1981-01-01), pages 155-158, XP055311417, CH ISSN: 0012-2823, DOI: 10.1159/000198627
- KHARENKO ET AL.: 'MUCOADHESIVE DRUG DELIVERY SYSTEMS (REVIEW' PHARMACEUTICAL CHEMISTRY JOURNAL vol. 43, no. 4, 2009, pages 200 - 208, XP055293467
- FRISHMAN ET AL.: 'Beta-Adrenergic Blockers in Systemic Hypertension Pharmacokinetic Considerations Related to the Current Guidelines' CLIN PHARMACOKINET vol. 41, no. 7, 2002, pages 505 - 516, XP008181141

## Description

The present invention is directed to nadolol for use in treating an inflammatory bowel disorder (IBD) in a human in need thereof, wherein the nadolol is formulated for release in the small intestine or large intestine of the human.

### BACKGROUND OF THE INVENTION

Gastrointestinal diseases include a broad range of disorders related to the digestive tract. For example, common gastrointestinal diseases include inflammation and ulcers of any part of the digestive tract such as the esophagus, stomach, or duodenum. A significant fraction of the world population experiences one or more gastrointestinal diseases at some time during their lives. For example, it is estimated that as many as 1.4 million people in the United States suffer from Crohn's disease. Such disorders impose a significant disease burden on the United States healthcare system, with an estimated cost of more than $1.7 billion annually for inflammatory bowel disorders alone. Therefore, there remains a significant need for the treatment and management of gastrointestinal diseases and other disorders mediated by related biological pathways.

Ulcerative colitis is a disease of the colon wherein it is ulcerated and a symptomatic patient may have diarrhea. UC is treated as an autoimmune disease with anti-inflammatory or immunosuppressive agents, including biological therapeutics that targeting specific components of the immune response. Patients may not respond such treatment.

Beta blockers including nadolol, timolol, harmalol, levobunolol, bisoprolol, carteolol, pindolol, metoprolol, acebutolol, propranolol, atenolol, sotalol, penbutolol, labetalol, pronetalol, oxprenolol, practolol, betaxolol, and dexpropranolol are known for being able to decrease heart rate and blood pressure. There is no teaching of beta blockers, including nadolol or timolol, as agents for treating ulcerative colitis. An article in Digestion 1981, 22(3), 155 discloses that timolol maleate was not effective in the treatment of irritable bowel syndrome. US 2005/143378 discusses the beta blocker timidol formulated for release in the rectum for use in methods of treating gastrointestinal diseases in humans.

Steroids such as budesonide, dexamethasone, hydrocortisone, methylprednisolone, prednisolone, prednisone and triamcinolone are known agents for treating ulcerative colitis. However, long-term usage of corticosteroids is often accompanied by numerous systemic side effects, such as osteoporosis, diabetes mellitus, systemic hypertension, psychiatric disorders and altered steroid metabolism.

### SUMMARY OF THE INVENTION

The invention provides nadolol for use in treating an inflammatory bowel disorder in a human in need thereof, wherein the beta blocker is formulated for release in a small intestine or a large intestine of the human.

The subject is a human. The gastrointestinal disease is inflammatory bowel disease such as Crohn's disease or colitis, for instance, ulcerative colitis. In some embodiments, the gastrointestinal disease is prevented from progressing in said subject. In some embodiments, the subject is not in need of treatment for hypertension, angina, migraine headache, irregular heartbeat, or Parkinson's disease.

In some embodiments, the nadolol results in an improvement in the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject.

In some embodiments, the administering of the compound occurs prior to, concurrent with, or after administration of an additional treatment regimen or therapeutic agent to said subject. The additional therapeutic agent is, for instance, a steroid or other anti-inflammatory drug. In some embodiments, the additional treatment regimen comprises traditional or laparoscopic surgery. In other embodiments, the additional treatment is a laxative or an anti-constipation agent. In other embodiments, the additional therapeutic agent is antacid or other acidic reducer.

In another aspect, the compound is administered via pH-dependent release, via microbially-triggered delivery, as a conjugate, via time-controlled delivery, via osmotically-regulated delivery, administered via pressure-controlled delivery, via multi matrix systems delivery, via bioadhesion delivery, or via multiparticulate delivery.

In another aspect, the compound is released preferentially in the small or large intestine. In some embodiments, at least 60%, 70%, 80%, or 90% of the administered dose of the compound is released.

In some embodiments of the composition provided herein, the compound is formulated for administration via pH-dependent release delivery, microbially-triggered delivery, time-controlled delivery, osmotically-regulated delivery, pressure-controlled delivery, multi matrix systems delivery, bioadhesion delivery, or multiparticulate delivery. The compound is formulated for release in the small or large intestine.

Also provided is nadolol for use in treating an inflammatory bowel disorder in a subject in need thereof, comprising administering to the subject a pharmaceutical composition, wherein the pharmaceutical composition is delivered by rectal administration. The pharmaceutical composition is, for example, chosen from the group consisting of enemas, rectal gels, rectal foams, rectal aerosols, suppositories, jelly suppositories, or retention enemas. In some embodiments, the pharmaceutical composition is a suppository or a rectal gel. In some embodiments, the pharmaceutical composition comprises less than 40, 20, 10, 8, 6, 4, or 2 mg of beta blocker. In some embodiments, the subject is administered less than 40, 20, 10, 8, 6, 4, or 2 mg of beta blocker daily. In some embodiments, the subject is administered less than 0.6, 0.4, 0.2, 0.1, 0.05, 0.02, or 0.01 mg of beta blocker per kg of the subject's body weight daily.

The subject is a human. The gastrointestinal disease is inflammatory bowel disease, Crohn's disease, or colitis, for instance, ulcerative colitis. In some embodiments, the gastrointestinal disease is prevented from progressing in said subject. In some embodiments, the administering step results in an improvement in the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject.

In some embodiments of the compound for use herein, the pharmaceutical composition comprises an amount of nadolol, which, when administered to the subject orally, is insufficient to result in an improvement in the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject. In other embodiments, the pharmaceutical composition comprises an amount of nadolol which is sufficient to result in an improvement in the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject. In some embodiments, the improvement results in a decrease of colonic score by at least 1, 2, 3, 4, or 5. In some embodiments, the improvement results in a decrease of colonic score by at least 3 or 4. In some embodiments, the improvement is a decrease in colonic score by 1 or 2. In some embodiments of the method, administration of the pharmaceutical composition results in a peak plasma level in the subject of 50, 40, 30, 20, or 10 ng/mL, for example, less than 50, 30 or 10 ng/mL.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIG. 1a shows the effect of oral ("PO") treatment with Nadolol on colon weight and length in a prophylactic TNBS rat colitis model. FIG. 1b shows the effect of intra-rectal ("IR") treatment with Nadolol on colon weight and length in a prophylactic TNBS rat colitis model.
FIG. 2a shows the effect of oral treatment with Nadolol on colonic parameters in a prophylactic TNBS rat colitis model. FIG. 2b shows the effect of intra-rectal treatment with Nadolol on colonic parameters in a prophylactic TNBS rat colitis model.
FIG. 3a shows the effect of oral treatment with Nadolol on colonic score in a prophylactic TNBS rat colitis model. FIG. 3b shows the effect of intra-rectal treatment with Nadolol on colonic score in a prophylactic TNBS rat colitis model.

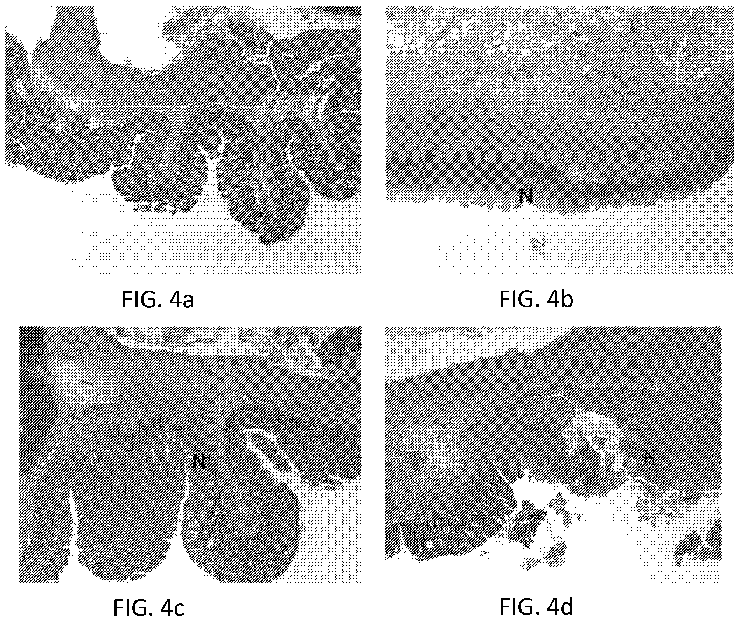

FIG. 4 shows magnified pictures of colons from prophylactic TNBS rat colitis model. FIG. 4a shows colon from an animal that was not given TNBS and has no lesions. FIG. 4b shows colon from a PO vehicle control animal (with the approximate mean summed score for the group) has severe necrosis (N) and inflammation. FIG. 4c shows colon from a diseased animal given 10 mpk of prednisolone (with the approximate mean summed score for the group) has very minimal necrosis ("N") and minimal inflammation. FIG 4d shows colon from a diseased animal given 60 mpk of Nadolol (with the approximate mean summed score for the group) which has moderate necrosis (N) and inflammation.

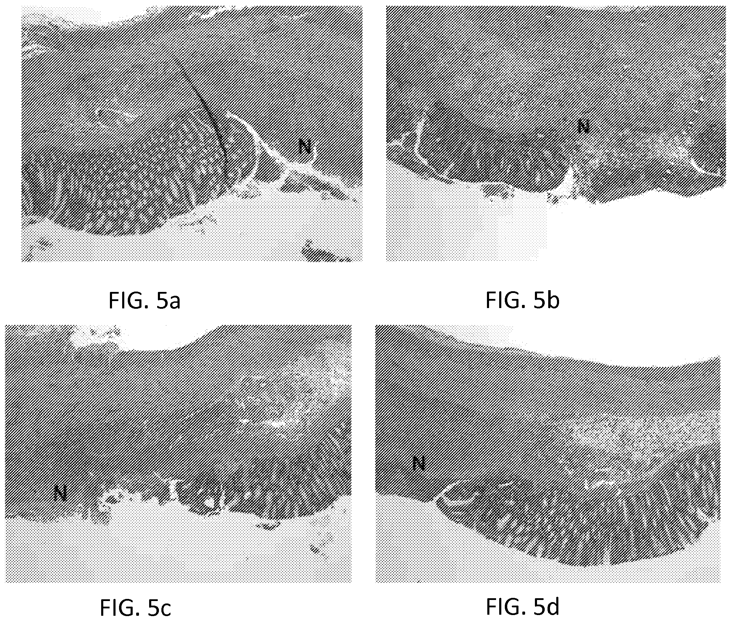

FIG. 5 shows magnified pictures of colons from prophylactic TNBS rat colitis model. FIG. 5a shows colon from a diseased animal given 120 mpk of Nadolol (with the approximate mean summed score for the group) which has mild necrosis (N) and moderate inflammation. FIG. 5b shows colon from an IR vehicle control animal (with the approximate mean summed score for the group) which has mild necrosis (N) and marked inflammation. FIG. 5c shows colon from a diseased animal given 60 mpk of Nadolol (with the approximate mean summed score for the group) which has moderate necrosis (N) and moderate inflammation. FIG. 5d shows colon from a diseased animal given 120 mpk of Nadolol (with the approximate mean summed score for the group) which has mild necrosis (N) and mild inflammation.
FIG. 6 shows mean histopathology scores in necrosis and inflammation for each treatment group in the prophylactic TNBS rat colitis model.
FIG. 7 shows necrosis ratio (mean plus/minus SE) for each treatment group in the prophylactic TNBS rat colitis model.
FIG. 8 shows summed mean histopathology score (mean plus/minus SE) for each treatment group in the prophylactic TNBS rat colitis model.
FIG. 9a shows the effect of oral treatment with Nadolol at various doses on colon weight and length in a prophylactic TNBS rat colitis model. FIG. 9b shows the effect of intra-rectal treatment with Nadolol at various doses on colon weight and length in a prophylactic TNBS rat colitis model.
FIG. 10a shows the effect of oral treatment with Nadolol at various doses on colonic parameters in a prophylactic TNBS rat colitis model. FIG. 10b shows the effect of intra-rectal treatment with Nadolol at various doses on colonic parameters in a prophylactic TNBS rat colitis model.
FIG. 11a shows the effect of oral treatment with Nadolol at various doses on colonic score in a prophylactic TNBS rat colitis model. FIG. 11b shows the effect of intra-rectal treatment with Nadolol at various doses on colonic score in a prophylactic TNBS rat colitis model.

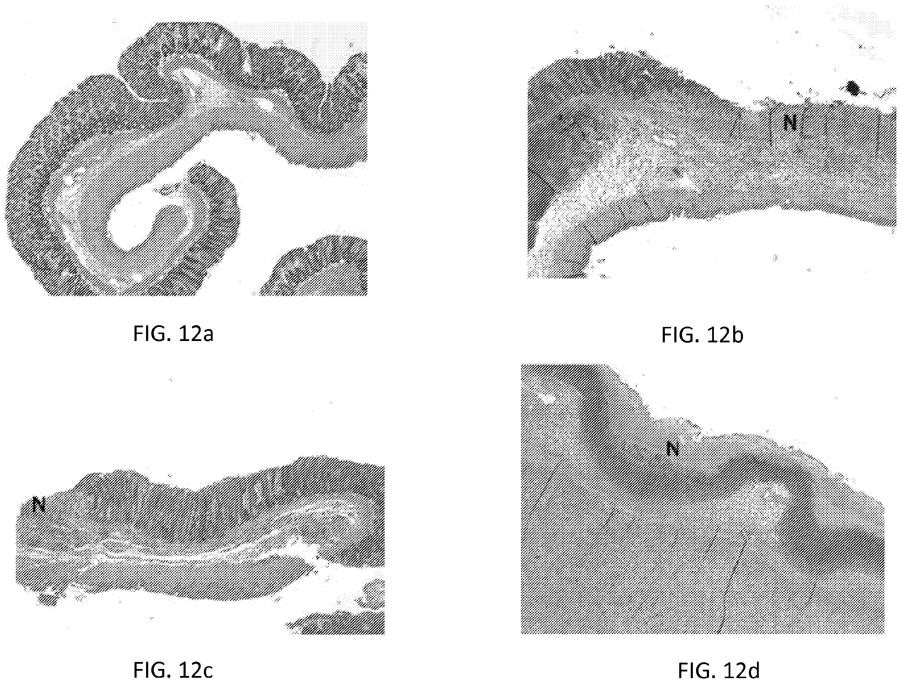

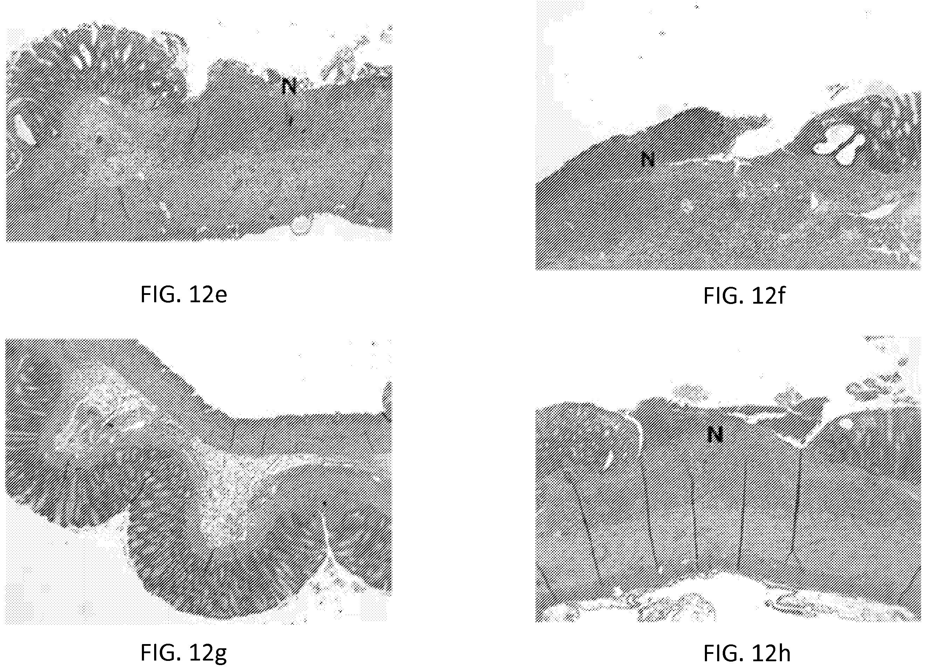

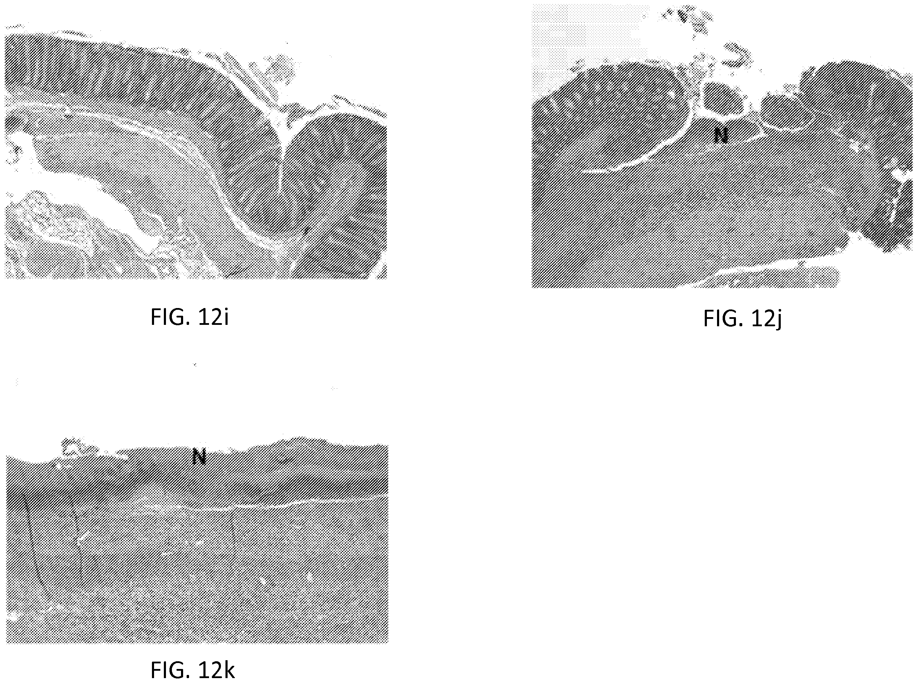

FIG. 12 shows magnified pictures of colons from prophylactic TNBS rat colitis model. FIG. 12a shows colon from an animal that was not exposed to TNBS and has no lesions.. FIG. 12b shows colon section from a PO vehicle control animal (with the approximate mean summed score for the group) which has marked necrosis (N) and inflammation. FIG. 12c shows colon from a diseased animal treated PO with 10 mpk of Prednisolone (with the approximate mean summed score for the group) which has minimal necrosis (N) and inflammation. FIG. 12d shows colon from a diseased animal treated PO with 6 mpk of Nadolol (with the approximate mean summed score for the group) which has severe necrosis (N) and mild inflammation. FIG. 12e shows colon from a diseased animal treated PO with 19 mpk of Nadolol (with the approximate mean summed score for the group) which has marked necrosis (N) and inflammation FIG. 12f shows colon section from a diseased animal treated PO with 60 mpk of Nadolol (with the approximate mean summed score for the group) which has severe necrosis (N) and inflammation. FIG. 12g shows colon section from a diseased animal treated PO with 120 mpk of Nadolol (with the approximate mean summed score for the group) which has no necrosis and mild inflammation. FIG. 12h shows colon section an IR vehicle control animal (with the approximate mean summed score for the group) which has moderate necrosis (N) and marked inflammation. FIG. 12i shows colon section from a diseased animal treated IR with 6 mpk of Nadolol (with the approximate mean summed score for the group) which has no necrosis and mild inflammation. FIG. 12j shows colon section from a diseased animal treated IR with 19 mpk of Nadolol (with the approximate mean summed score for the group) which has mild necrosis (N) and moderate inflammation. FIG. 12k shows colon section from a diseased animal treated IR with 60 mpk of Nadolol (with the approximate mean summed score for the group) which has severe necrosis and inflammation.
FIG. 13 shows mean histopathology scores in necrosis and inflammation for each PO treatment group in a prophylactic TNBS rat colitis model.
FIG. 14 shows mean histopathology scores in necrosis and inflammation for each IR treatment group in a prophylactic TNBS rat colitis model
FIG. 15 shows summed mean histopathology score (mean plus/minus SE) for each PO treatment group in a prophylactic TNBS rat colitis model.
FIG. 16 shows summed mean histopathology score (mean plus/minus SE) for each IR treatment group in a prophylactic TNBS rat colitis model.
FIG. 17 shows the necrosis ratio to total length (mean plus/minus SE) for each PO treatment group in a prophylactic TNBS rat colitis model.
FIG. 18 shows the necrosis ratio to total length (mean plus/minus SE) for each IR treatment group in a prophylactic TNBS rat colitis model.

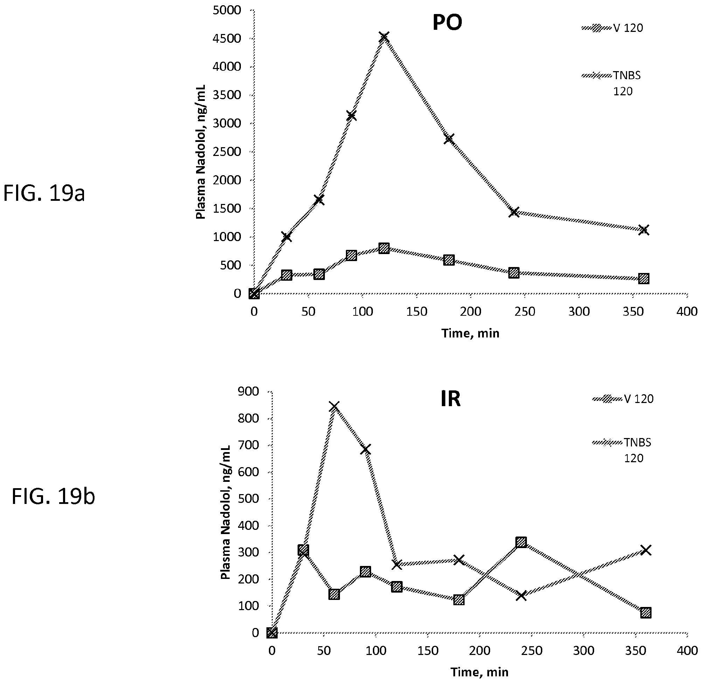

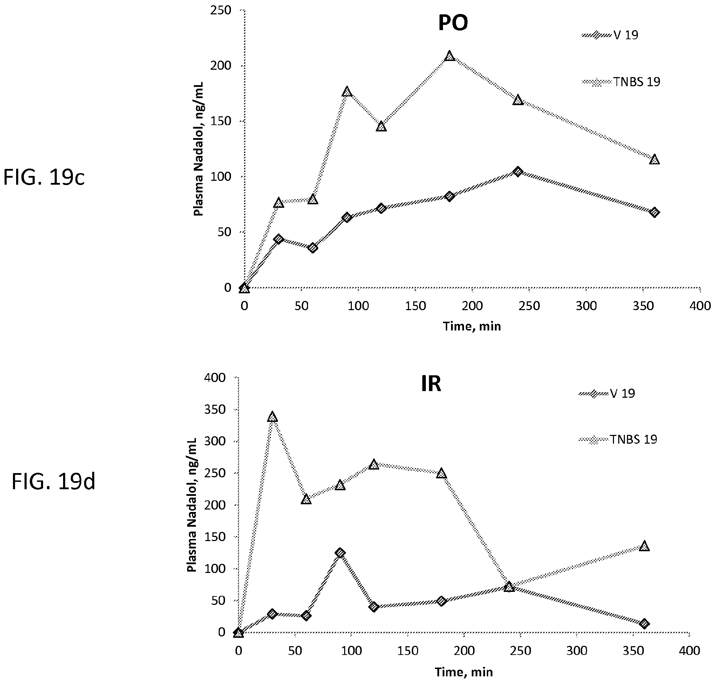

FIG. 19a shows the exposure of Nadolol (Plasma Nadolol, ng/mL) over time for a diseased rat TNBS rat colitis model dosed with 120 mpk Nadolol ("TNBS 120") PO and a healthy rat ("V 120") dosed with 120 mpk Nadolol PO. FIG. 19b shows the exposure of Nadolol (Plasma Nadolol, ng/mL) over time for a diseased rat TNBS rat colitis model dosed with 120 mpk Nadolol ("TNBS 120") IR and a healthy rat ("V 120") dosed with 120 mpk Nadolol IR. FIG. 19c shows the exposure of Nadolol (Plasma Nadolol, ng/mL) over time for a diseased rat TNBS rat colitis model dosed with 19 mpk Nadolol ("TNBS 19") PO and a healthy rat ("V 19") dosed with 19 mpk Nadolol PO. FIG. 19d shows the exposure of Nadolol (Plasma Nadolol, ng/mL) over time for a diseased rat TNBS rat colitis model dosed with 19 mpk Nadolol ("TNBS 19") IR and a healthy rat ("V 19") dosed with 19 mpk Nadolol IR
FIG 20 shows the average colonic score of rats in a TNBS colitis model treated with various beta blockers oral administration (PO) versus prednisolone or vehicle (+TNBS).
FIG 21 shows the average colonic score of rats in a TNBS colitis model treated with various beta blockers Intra-rectal administration (IR) versus prednisolone or vehicle (+TNBS).
FIG 22 shows the histopathology average score for colonic necrosis, colonic inflammation, and sum for rats in a TNBS colitis model treated with various beta blockers Intra-rectal administration (IR) versus prednisolone or vehicle (+TNBS) or healthy control (No TNBS).
FIG 23 shows the effect of treatment on the histopathology ratio score for rats in a TNBS colitis model treated with various beta blockers Intra-rectal administration (IR) versus prednisolone or vehicle (+TNBS) or healthy control (No TNBS).

### DETAILED DESCRIPTION OF THE INVENTION

### Definitions

As used above, and throughout the description of the invention, the following abbreviations, unless otherwise indicated, shall be understood have the following meanings.

The term "gastrointestinal disease" as used herein refers to all diseases or disorders that pertain to the gastrointestinal tract. This includes diseases of the esophagus, stomach, first, second, and third part of the duodenum, jejunum, ileum, the ileo-cecal complex, large intestine (ascending, transverse, and descending colon), sigmoid colon, rectum, and anus.

As used herein, "agent" or "biologically active agent" refers to a biological, pharmaceutical, or chemical compound or other moiety. Examples include simple or complex organic or inorganic molecule, a peptide, a protein, an oligonucleotide, an antibody, an antibody derivative, antibody fragment, a vitamin derivative, a carbohydrate, a toxin, or a chemotherapeutic compound. Various compounds can be synthesized, for example, small molecules and oligomers (e.g., oligopeptides and oligonucleotides), and synthetic organic compounds based on various core structures.

The term "beta blocker" (can also be called a β-blocker, beta-adrenergic blocking agent, beta antagonist, beta-adrenergic antagonists, beta-adrenoreceptor antagonists, or beta adrenergic receptor antagonists) refers to a compound that binds to a beta receptor. Some beta blockers are members of the class of drugs that target at least one beta receptor. Beta blockers can have high affinity for one or more beta receptors. For instance, some beta blockers can bind multiple beta receptors with similar affinity. Other beta blockers selectively bind one beta receptor with higher affinity than other beta receptors. Examples of a beta blocker include nadolol, timolol, harmalol, levobunolol, bisoprolol, alprenolol, carteolol, pindolol, metoprolol, acebutolol, S-propranolol, (-)-atenolol, sotalol, propranolol, R-atenolol, penbutolol, labetalol, pronetalol, oxprenolol, practolol, betaxolol, and dexpropranolol.

The term "effective amount", "effective therapeutic amount" or "therapeutically effective amount" refers to that amount of compound or biological agent that is sufficient to effect the intended applications, including clinical results such as reducing bleeding, reducing inflammation, reducing diarrhea, reducing constipation, inhibiting ulcer formation, restoring digestive function, decreasing symptoms resulting from the disease, increasing the quality of life of those suffering from the disease, enhancing the effect of another medication, delaying the progression of the disease, and/or prolonging survival of individuals. The therapeutically effective amount will vary depending upon the subject and disease condition being treated, the weight and age of the subject, the severity of the disease condition, the manner of administration and the like, which can readily be determined by one of ordinary skill in the art.

The term "pharmaceutically acceptable salts" refers to the relatively non-toxic, inorganic and organic acid addition salts of compounds of the present invention. These salts can be prepared in situ during the final isolation and purification of the compounds. In particular, acid addition salts can be prepared by separately reacting the purified compound in its free base form with a suitable organic or inorganic acid and isolating the salt thus formed. Exemplary acid addition salts include the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, sulphamates, malonates, salicylates, propionates, methylene-bis-beta-hydroxynaphthoates, gentisates, isethionates, di-p-toluoyltartrates, methanesulphonates, ethanesulphonates, benzenesulphonates, p-toluenesulphonates, cyclohexylsulphamates and quinateslauryl-sulphonate salts. See, for example S. M. Berge, et al., "Pharmaceutical Salts," J. Pharm. Sci., 66, 1-19 (1977).

The term "inhibit," as used herein, refers to the ability of a compound or any agent to reduce or impede a described function, level, activity, synthesis, release, binding, etc., based on the context in which the term "inhibit" is used. The term "inhibit" is used interchangeably with "reduce," "block," and "decrease."

As used herein, "treatment" or "treating," or "palliating" or "ameliorating" are used interchangeably herein. These terms refer to an approach for obtaining beneficial or desired results including therapeutic benefit and/or a prophylactic benefit. By therapeutic benefit is meant eradication, amelioration or stabilization of the underlying disorder being treated (e.g., in the context of many gastrointestinal diseases, complete or substantially complete mucosal healing). Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made. For purposes of this invention, beneficial or desired clinical results include one or more of the following: reducing, decreasing symptoms resulting from the disease, increasing the quality of life of those suffering from the disease, decreasing the dose of other medications required to treat the disease, enhancing the effect of another medication, delaying the progression of the disease, and/or prolonging survival of individuals. Treatment includes preventing the disease, that is, causing the clinical symptoms of the disease not to develop by administration of a protective composition prior to the induction of the disease, suppressing the disease, that is, causing the clinical symptoms of the disease not to develop by administration of a protective composition after the inductive event but prior to the clinical appearance or reappearance of the disease, inhibiting the disease, that is, arresting the development of clinical symptoms by administration of a protective composition after their initial appearance, preventing re-occurring of the disease and/or relieving the disease, that is, causing the regression of clinical symptoms by administration of a protective composition after their initial appearance.

The terms "subject," "individual" or "patient" are used interchangeably herein, and refer to a vertebrate, for example a mammal, including a human. Mammals include murines, simians, humans, farm animals, sport animals, and pets. Tissues, cells and their progeny of a biological entity obtained *in vitro* or cultured *in vitro* are also encompassed.

"Signal transduction" is a process during which stimulatory or inhibitory signals are transmitted into and within a cell to elicit an intracellular response. A modulator of a signal transduction pathway refers to a compound, which modulates the activity of one, or more cellular proteins mapped to the same specific signal transduction pathway. A modulator may augment (agonist) or suppress (antagonist) the activity of a signaling molecule.

The term "selective inhibition" or "selectively inhibit" as referred to a biologically active agent refers to the agent's ability to preferentially reduce the target signaling activity as compared to off-target signaling activity, via direct or interact interaction with the target.

A "therapeutic effect" as used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

The term "susceptibility" or "susceptible" as used herein, refers to a subject determined to be at risk for having a disease condition. Such a determination may be based on an analysis including, (i) familial disease history, (ii) a genotypic characteristic of the subject, and/or (iii) a phenotypic characteristic of the subject.

The term "co-administration," "administered in combination with," and their grammatical equivalents, as used herein, encompasses administration of two or more agents to a subject so that both agents and/or their metabolites are present in the subject at the same time. Co-administration includes simultaneous administration in separate compositions, administration at different times in separate compositions, or administration in a composition in which both agents are present.

The term "*in vivo*" refers to an event that takes place in a subject's body.

The term "*in vitro*" refers to an event that takes places outside of a subject's body. For example, an *in vitro* assay encompasses any assay run outside of a subject assay. *In vitro* assays encompass cell-based assays in which cells alive or dead are employed. *In vitro* assays also encompass a cell-free assay in which no intact cells are employed.

### Embodiments

### Composition embodiments

The beta blocker of the present invention is nadolol.

Further provided herein are pharmaceutically acceptable compositions comprising nadolol.

For example, the composition is formulated for administration via pH-dependent release delivery, microbially-triggered delivery, time-controlled delivery, osmotically-regulated delivery, pressure-controlled delivery, multi matrix systems delivery, bioadhesion delivery, or multiparticulate delivery. The composition of the invention is formulated for release in the small or large intestine.

In some examples, the compositions disclosed herein may be delivered to any part of the digestive tract, including but not limited to the colon, the intestine, the stomach, or the esophagus. For example, a composition is delivered primarily to the colon via a colon-specific drug delivery method. Possible colon-specific drug delivery methods include the following: pH-dependent release, microbially-triggered drug delivery, conjugates, time-controlled delivery, osmotically-regulated delivery, pressure-controlled delivery, multi matrix delivery systems, bioadhesion, microparticulate or nanoparticulate delivery. The drug delivery method may include any combination of colon-specific drug delivery methods. For example, a compound may be formulated to minimize release of the drug in the stomach, small intestine, and/or upper GI tract and to promote release of the drug in the colon. The route of administration may be topical, enteral, parenteral, or a combination thereof. Enteral administration routes comprise a non-local administration of the drug via the digestive tract. Common enteral routes include oral, rectal, sublingual, sublabial, buccal, gastric feeding tube, duodenal feeding tube, or a combination thereof.

The compound may be coated, in some examples, with a pH-dependent polymer that inhibits or minimizes release of the drug in the stomach, small intestine, and/or upper GI tract and promotes release of the drug in the colon. The drug core may include tablets, capsules, pellets, granules, microparticles, nanoparticles, or a combination thereof. A suitable pH-dependent polymer may be a derivative of acrylic acid or cellulose. Examples of suitable pH-dependent polymers include : hydroxypropyl methylcellulose phthalate, hydroxypropyl methylcellulose acetate succinate, acrylic polymers, acrylic copolymers, methyl methacrylate-methacrylic acid copolymers, cellulose acetate phthalate, cellulose acetate succinate, cellulose acetate trimellate, Eudragit® L100, Eudragit® L100-55, Eudragit® S100, Eudragit® L-30D, Eudragit® FS 30D, hydroxypropyl methylcellulose phthalate 50, hydroxypropyl methylcellulose phthalate 55, or any combination thereof.

The drug compound may be embedded in a polymer matrix, wherein the polymer is pH-dependent. The drug delivery system may comprise Eduracol® technology, wherein a drug core is enclosed by multiple layers of Eudragit®. The drug delivery may comprise enclosure of a drug compound by multiple layers of pH-dependent polymers. The timing of the drug release may be controlled by the thickness and composition of the multiple layers surrounding the drug core.

The drug compound may be formulated to comprise a biodegradable polymer that reacts with the microflora or enzymes present in the colon, thereby facilitating delivery of the drug to the colon and inhibiting release of the drug in the stomach or small intestine. The drug compound may be coated with a biodegradable polymer. The drug compound may be embedded in biodegradable polymeric matrices or hydrogels. The drug delivery system may comprise both biodegradable polymers and pH-dependent polymers. The biodegradable polymer may be selected to react with a targeted colonic bacteria or enzyme. Examples of enzymes that a biodegradable polymer may react with include azoreductase, bacterial hydrolase glycosidase, esterase andpolysaccharidase.,For example, biodegradable polymers containing azoaromatic linkages may react with azoreductase. Examples of suitable biodegradable polymers include: azo polymers, polyurethane containing azo aromatic groups, azo polymers using styrene-2-hydroxyethyl methacrylate and divinyl azobenzene as a cross linker, copolymers of 2-hydroxyethyl methacrylate with bis(methacryloylamino)azo benzene as a cross linker, terapolymers of methyl methacrylate, 2-hydroxyethyl methacrylate and methacrylic acid with N,N-bis[(methacryloyloxyethyl)oxy(carbonylamino)]azo benzene, divinyl azobenzene, and bis(methacryloylamino)azobenzene as a cross linker, poly(ether-ester) azopolymers, urethanes containing azo aromatic linkages in the backbone, polygalactomannans in polymethacrylate solutions, inulin and copolymers of acrylic acid esters, pectin and ethyl cellulose, glutaraldehyde cross-linked dextran, poly(-caprolactone), polylactic acid, poly(lactic-co-glycolic acid), polysaccharides, amylose, guar gum, pectin, chitosan, inulin, cyclodextrin, chondroitin sulphate, dextran, locust bean gum, arabinogalactan, chondroitin sulfate, xylan, calcium pectinate, pectin/chitosan mixtures, amidated pectin, or any combination thereof.

In addition, a formulation of the drug compound may comprise a biodegradable matrix, which comprises cross-linked biodegradable polymers. The biodegradable polymers may be cross-linked to each other and added to the drug compound or the formulation. The biodegradable polymer matrix may comprise a synthetic hydrogel. The biodegradable polymer matrix may comprise a synthetic hydrogel based on N-substituted acrylamide. Additional hydrogels include hydrogels synthesized by cross-linking N-tert-butylacrylamide with acrylic acid or copolymerization of 2-hydroxyethyl methacrylate and 4-methacryloyloxyazo benzene.

The drug compound may be delivered via a time-controlled delivery system. An example of a suitable time-controlled delivery system is the Pulsincap® system, or a variant thereof. The time-controlled delivery system may further comprise pH-dependent systems, microbially-triggered delivery systems, or a combination thereof. The time-controlled system may comprise a water insoluble capsule body enclosing a drug reservoir. The capsule body may be closed at one end with a hydrogel plug. The hydrogel plug may comprise swellable polymers, erodible compressed polymers, congealed melted polymers, enzymatically controlled erodible polymers, or a combination thereof. The swellable polymers may include polymethacrylates. The erodible compressed polymers may include hydroxypropyl methylcellulose, polyvinyl alcohol, polyvinyl acetate, polyethylene oxide, or a combination thereof. The congealed melted polymers may include: saturated polyglycolated glycerides, glyceryl monooleate, or a combination thereof. The enzymatically controlled erodible polymers may include polysaccharides, amylose, guar gum, pectin, chitosan, inulin, cyclodextrin, chondroitin sulphate, dextrans, locust bean gum, arabinogalactan, chondroitin sulfate, xylan, calcium pectinate, pectin/chitosan mixtures, amidated pectin, or any combination thereof.

An additional example of a suitable time-controlled delivery system is Time Clock®, or a variant thereof, wherein a solid dosage form is coated with a hydrophobic surfactant layer to which a water-soluble polymer is added.

The time-controlled delivery system may comprise a capsule, which further comprises an organic acid. The organic acid may be filled into the body of a hard gelatine capsule. The capsule may be coated with multiple layers of polymers. The capsule may be coated first with an acid soluble polymer, such as Eudragit® E, then with a hydrophilic polymer, such as hydroxypropyl methylcellulose, and finally with an enteric coating, such as Eudragit® L.

An additional example of a suitable time-controlled delivery system is the Chronotropic® system, or a variant thereof, which comprises a drug core that is coated with hydroxypropyl methylcellulose and an outer enteric film.

An additional example of a suitable time-controlled delivery system is the CODES™ system, or a variant thereof. The time-controlled delivery system may comprise a capsule body, which may house a drug-containing tablet, an erodible tablet, a swelling expulsion excipient, or any combination thereof. The capsule may comprise an ethyl cellulose coat. The time-controlled delivery system may comprise two different sized capsules, one inside of the other. The space between the capsules may comprise a hydrophilic polymer. The drug-containing core may be housed within the inner capsule. The drug delivery system may comprise an impermeable shell, a drug-containing core, and erodible outer layers at each open end. When the outer layers erode, the drug is released.

The compound may be delivered to the colon via an osmotically-regulated delivery system. An example of a suitable osmotically-regulated delivery system is the OROS® Push-Pull™ osmotic pump technology, or a variant thereof. The osmotically controlled drug delivery system may comprise a semipermeable coat. The system may further comprise a bi-layer core containing two component layers within a compartment formed by a semipermeable wall having an exit means for release of the drug. In the bi-layer core, one layer contains the drug compound and the second layer contains osmotically active compounds. The two layers may be compressed into bi-layer tablet cores before the semipermeable membrane and exit means are applied. The bi-layer tablet cores may be longitudinally compressed with a different layer at each end. Osmotically active components includeosmopolymers, which are polymers that have relatively large molecular weights and swell when exposed to fluid. The layer containing the osmotically active components may comprise one or more of the following compounds: poly(ethylene)oxide, sodium chloride, poly(vinylpyrrolidone), red ferric oxide, magnesium stearate, butylated hydroxyl toluene, and hydroxypropyl methylcellulose. The semipermeable membrane may comprise cellulose acetate, poly(ethylene glycol), or a combination thereof.

Alternatively, the osmotically-regulated drug delivery system may comprise a trilayer core containing three component layers within a compartment formed by a semipermeable membrane and comprising an exit means for release of the drug. In the trilayer core, the first and second layers contain the drug compound, the third layer comprises the osmotically active components. The first and second layers may further comprise one or more of the following compounds: poly(ethylene)oxide, sodium chloride, poly(vinylpyrrolidone), red ferric oxide, magnesium stearate, butylated hydroxyl toluene, and hydroxypropyl methylcellulose. The third layer comprises osmopolymers. The semipermeable membrane may comprise cellulose acetate, poly(ethylene glycol), or a combination thereof.

An additional example of a suitable osmotically-regulated delivery system is the PORT® system, or a variant thereof. The osmotically-regulated drug delivery system may comprise a gelatin capsule coated with a semipermeable membrane housing an insoluble plug, an osmotically active agent, and the drug compound. The semipermeable membrane may comprise cellulose acetate, poly(ethylene glycol), or a combination thereof. The osmotically-regulated drug delivery system may comprise a water-insoluble capsule shell, which houses a drug containing compartment, an osmotic core, a rigid barrier layer, a semipermeable cap, an insoluble coat on the capsule, or any combination thereof.

The pressure-controlled delivery system may comprise a capsule that is fabricated from a water insoluble material, such as ethyl cellulose. Suitable pressure controlled colon-delivery capsules withstand the luminal pressures of the stomach and small intestine, but release the drug under the pressurized conditions in the colon. The capsule may comprise two coatings. The capsule may comprise an insoluble coating on the inside wall of the capsule. The capsule may comprise an insoluble waxy plug that seals the top of the insoluble coat. The capsule may be coated with an enteric coating.

The drug delivery system may comprise the drug compound inglobulated in multiple matrices. An example of a suitable multi matrix delivery system is the MMX® delivery system (Cosmo Pharmaceuticals), or a variant thereof. The matrices may include, but are not limited to, amphiphilic, lipophilic and hydrophilic matrices. The multi matrix may be coated with a pH-resistant coating, biodegradable polymeric coating, or some combination thereof. The amphiphilic matrix may comprise polar lipids of type I or II, lecithin, phosphatidylcholine, phosphatidylethanolamine, polyethylene glycols, ceramides, glycol alkyl ether, diethylene glycol monomethyl ether, or a combination thereof. The lipophilic matrix may comprise C8-C20 fatty acids with glycerol, unsaturated or hydrogenated alcohols or fatty acids, salts, esters or amide derivatives thereof, waxes, ceramides, cholesterol derivatives, or a combination thereof. The hydrophilic matrix may comprise hydrophilic water-swellable agents, known as hydrogels. Suitable hydrogels include hydroxypropylcellulose, acrylic or methacrylic acid polymers or copolymers, alkylvinyl polymers, hydroxyalkyl celluloses, caroxyalkyl celluloses, polysaccharides, dextrins, pectins, starches and derivatives, natural or synthetic gums, alginic acid, or some combination thereof. The pH-resistant coating may comprise acrylic copolymers, methacrylic acid polymers or copolymers, cellulose derivatives, or a combination thereof.

In some multi matrix systems, the drug compound is embedded in biodegradable matrices. In some multi matrix systems, the drug compound is embedded in pH-dependent matrices. In some multi matrix systems, the drug compound is embedded in pH-dependent and biodegradable matrices.

The drug delivery system may comprise a bioadhesive polymer that selectively adheres to the colonic mucosa. Bioadhesive polymers facilitate extended contact of the drug with the tissues of a subject. The high molecular weight of these polymers readily swell in body fluids, providing a large adhesive surface for maximum contact with the mucous layer of such tissues as the colon. These polymers have been shown to increase drug bioavailability due to the lengthened period of time in which the drug is in contact with the absorbing tissue. Bioadhesive systems may comprise polymers such as polycarbophils, polyurethanes, polyethylene oxide-polypropylene oxide copolymers, or some combination thereof. Bioadhesive that could be used with Formula I include Carbopol® polymers, Pemulen™ polymeric emulsifiers and Noveon® polycarbophils or variants thereof.

Examples of suitable multiparticulate drug delivery systems include Diffucaps®, Diffutab®, Orbexa®, Eurand Minitabs®, Microcaps®, or a variant thereof. The drug delivery system may comprise multiparticulate beads, which are comprised of multiple layers of the drug compound, excipients, and release-controlling polymers. The multiparticulate beads may comprise an organic acid or alkaline buffer. The multiparticulate beads may comprise a solid solution of the drug compound and crystallization inhibitor. The drug delivery system may comprise a matrix tablet containing water-soluble particles and the drug compound. The matrix tablet may further comprise hydrophilic and hydrophobic polymers.In some multiparticulate delivery systems, particles in the micron size range are used. In some multiparticulate delivery systems, nanoparticle colloidal carriers composed of natural or synthetic polymers are used.

Compositions comprising nadolol can dissolve or disintegrate rapidly in a subject's mouth when administered orally. Therefore, a pleasant taste can be important for ensuring compliance with a therapeutic regimen. Taste-masking technologies provide the benefit of an agreeable taste and an absence of grit residue left behind in the mouth, thus leaving the subject with a good mouth feel. Examples of suitable taste-masking technologies that can be used with Formula I include, but are not limited to: Zydis ®, Quicksolv®, Lyoc®, NanoCrystal®, AdvaTab®, OraSolv®, DuraSlov®, FlashDose®, Flash Melt®, Flashtab®, OraQuick®, Pharmburst®, Frosta® and WOWTAB®.

Therapeutically-effective dosages vary in some embodiments when the nadolol is used in treatment combinations. Methods for experimentally determining therapeutically-effective dosages of drugs and other agents for use in combination treatment regimens include the use of metronomic dosing, i.e., by providing more frequent, lower doses in order to minimize toxic side effects. Combination treatment regimens encompass treatment regimens in which administration of a compound described herein is initiated prior to, during, or after treatment with a second agent described above, and continues until any time during treatment with the second agent or after termination of treatment with the second agent. Such regimens also include treatments in which a compound described herein and the second agent being used in combination are administered simultaneously or at different times and/or at decreasing or increasing intervals during the treatment period. Combination treatments further include periodic treatments that start and stop at various times to assist with the clinical management of the patient. For example, a compound described herein in the combination treatment is administered weekly at the onset of treatment, decreasing to biweekly, and decreasing further as appropriate.

In any combination treatment regimen, a therapeutically effective amount of the nadolol may be administered in an amount which is less therapeutically effective if administered alone. For any of the classes of additional therapeutic agents mentioned herein that are used in combination treatments, the additional therapeutic agent is optionally administered in an amount which would be less therapeutically effective if administered alone. By way of example only, if an analgesic is used in a combination treatment, the analgesic is administered in an amount which is less therapeutically effective if administered alone.

In any case, the therapeutic agents that make up a combination treatment (at least one of which is nadolol) are administered in any order or even simultaneously. If administered simultaneously, the multiple therapeutic agents are optionally provided in a single, unified form, or in multiple forms (by way of example only, either as a single pill or as two separate pills). One of the therapeutic agents is optionally given in multiple doses, or both are given as multiple doses. If not simultaneous, the timing between the multiple doses optionally varies from more than zero weeks to less than four weeks. In addition, the combination methods, compositions and formulations are not to be limited to the use of only two agents, the use of multiple therapeutic combinations are also envisioned.

In addition, nadolol is also used in combination with procedures that provide additional or synergistic benefit to the patient. By way of example only, patients are expected to find therapeutic and/or prophylactic benefit in the methods described herein, wherein pharmaceutical composition of the invention and/or combinations with other therapeutics are combined with genetic testing to determine whether that individual is a carrier of a mutant gene that is known to be correlated with certain diseases or conditions.

Nadolol and combination therapies according to the invention are administered before, during and/or after the occurrence of a disease or condition, and the timing of administering the composition containing a compound optionally varies. Thus, for example, the compounds are used as a prophylactic and are administered continuously to subjects with a propensity to develop conditions or diseases in order to prevent the occurrence of the disease or condition. The compounds and compositions are optionally administered to a subject during or as soon as possible after the onset of the symptoms. For example, the administration of the compounds is initiated within the first 48 hours of the onset of the symptoms, for example within the first 48 hours of the onset of the symptoms, or within the first 6 hours of the onset of the symptoms, or within 3 hours of the onset of the symptoms. The initial administration is via any route practical, such as, for example, an intravenous injection, a bolus injection, infusion over 5 minutes to about 5 hours, a pill, a capsule, transdermal patch, buccal delivery, and the like, or combination thereof. A compound is administered, for example, as soon as is practicable after the onset of a disease or condition is detected or suspected, and for a length of time necessary for the treatment of the disease, such as, for example, from about 1 month to about 3 months. The length of treatment potentially varies for each subject, but the length is determined using the known criteria. For example, the compound or a formulation containing the compound is administered for at least 2 weeks, about 1 month to about 5 years, or from about 1 month to about 3 years.

Suitable routes of administration include oral, intravenous, rectal, aerosol, parenteral, ophthalmic, pulmonary, transmucosal, transdermal, vaginal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

In certain examples, a compound as described herein is administered in a local rather than systemic manner, for example, via injection of the compound directly into an organ, often in a depot preparation or sustained release formulation. In specific examples, long acting formulations are administered by implantation (for example subcutaneously or intramuscularly) or by intramuscular injection. Furthermore, in some examples, the drug is delivered in a targeted drug delivery system, for example, in a liposome coated with organ-specific antibody. In such examples, the liposomes are targeted to and taken up selectively by the organ. In some embodiments, the compound as described herein is provided in the form of a rapid release formulation, in the form of an extended release formulation, or in the form of an intermediate release formulation. In some examples, the compound described herein is administered topically.

In some examples and embodiments, the compounds described herein are formulated into pharmaceutical compositions. In specific embodiments, pharmaceutical compositions are formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995), Hoover, John E., Remington's Pharmaceutical Sciences, Mack Publishing Co., Easton, Pennsylvania 1975, Liberman, H.A. and Lachman, L., Eds., Pharmaceutical Dosage Forms, Marcel Decker, New York, N.Y., 1980, and Pharmaceutical Dosage Forms and Drug Delivery Systems, Seventh Ed. (Lippincott Williams & Wilkins1999).

Provided herein are pharmaceutical compositions comprising nadolol, and a pharmaceutically acceptable diluent(s), excipient(s), or carrier(s). In certain embodiments, nadolol is administered as pharmaceutical compositions in which the nadolol is mixed with other active ingredients, as in combination therapy. Encompassed herein are all combinations of actives set forth in the combination therapies section below and throughout this disclosure..

A pharmaceutical composition, as used herein, refers to a mixture of a nadolol, with other chemical components, such as carriers, stabilizers, diluents, dispersing agents, suspending agents, thickening agents, and/or excipients. In certain embodiments, the pharmaceutical composition facilitates administration of the compound to an organism. In some embodiments, practicing the methods of treatment or use provided herein, therapeutically effective amounts of the compound, provided herein are administered in a pharmaceutical composition to a mammal having a disease or condition to be treated. The mammal is a human. In certain embodiments, therapeutically effective amounts vary depending on the severity of the disease, the age and relative health of the subject, the potency of the compound used and other factors. The compounds described herein are used singly or in combination with one or more therapeutic agents as components of mixtures.

In some embodiments, the nadolol is formulated in an aqueous solution. In specific embodiments, the aqueous solution is selected from, by way of example only, a physiologically compatible buffer, such as Hank's solution, Ringer's solution, or physiological saline buffer. In some embodiments, the compound is formulated for transmucosal administration. In specific embodiments, transmucosal formulations include penetrants that are appropriate to the barrier to be permeated. In some embodiments wherein the compounds described herein are formulated for other parenteral injections, appropriate formulations include aqueous or nonaqueous solutions. In specific embodiments, such solutions include physiologically compatible buffers and/or excipients.

In some embodiments, compounds described herein are formulated for oral administration. For clarity, formulations intended for colonic delivery can be administered by oral administration. Compounds described herein, including compounds of Formula I, are formulated by combining the active compounds with, e.g., pharmaceutically acceptable carriers or excipients. In various embodiments, the compounds described herein are formulated in oral dosage forms that include, by way of example only, tablets, powders, pills, dragees, capsules, liquids, gels, syrups, elixirs, slurries, suspensions and the like.

In certain embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol, cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose, or others such as: polyvinylpyrrolidone (PVP or povidone) or calcium phosphate. In specific embodiments, disintegrating agents are optionally added. Disintegrating agents include, by way of example only, cross-linked croscarmellose sodium, polyvinylpyrrolidone, agar, or alginic acid or a salt thereof such as sodium alginate.

In some embodiments, dosage forms, such as dragee cores and tablets, are provided with one or more suitable coating. In specific embodiments, concentrated sugar solutions are used for coating the dosage form. The sugar solutions optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In certain embodiments, therapeutically effective amounts of at least one of the compounds described herein are formulated into other oral dosage forms. Oral dosage forms include push-fit capsules made of gelatin, as well as soft, sealed capsules made of gelatin and a plasticizer, such as glycerol or sorbitol. In specific embodiments, push-fit capsules contain the active ingredients in admixture with one or more filler. Fillers include, by way of example only, lactose, binders such as starches, and/or lubricants such as talc or magnesium stearate and, optionally, stabilizers. In some embodiments, soft capsules, contain one or more active compound that is dissolved or suspended in a suitable liquid. Suitable liquids include, by way of example only, one or more fatty oil, liquid paraffin, or liquid polyethylene glycol. In addition, stabilizers are optionally added.

In some embodiments, the compounds described herein are formulated for parental injection, including formulations suitable for bolus injection or continuous infusion. In specific embodiments, formulations for injection are presented in unit dosage form (*e*.*g*., in ampoules) or in multi-dose containers. Preservatives are, optionally, added to the injection formulations. In some embodiments, the pharmaceutical composition, are formulated in a form suitable for parenteral injection as sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active compounds in water-soluble form. In additional embodiments, suspensions of the active compounds are prepared as appropriate oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In certain specific embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the compounds to allow for the preparation of highly concentrated solutions. Alternatively, in some embodiments, the active ingredient is in powder form for constitution with a suitable vehicle, *e*.*g*., sterile pyrogen-free water, before use.

In some examples and embodiments, nadolol is formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone or PEG. In suppository forms of the compositions, a low-melting wax such as a mixture of fatty acid glycerides, optionally in combination with cocoa butter is first melted.

For example, the nadolol (e.g., in powder form) is typically dispersed in a suppository base, such as hard fat. The suppository base can be an oily or fatty base. Conventional suppository bases which may be employed include theobroma oil, hard fats, glycerides of fatty acids, glycerol-gelatin bases, and mixtures thereof. Suitable hard fat bases include esterified mixtures of mono-, di- and triglycerides which are obtained by esterification of fatty acids (European Pharmacopoeia, 3rd edition 1997, Deutscher Apotheker Verlag Stuttgart. p. 1022; The United States Pharmacopoeia, USP 23, NF18). Such hard fats are commercially available, for example, under the name Witepsol® (e.g. Witepsol® H12 and H15). A preferred suppository base is hard fat (e.g., hard fat NF). Preferred hard fat bases include hard fats containing a mixture of mono-, di- and triglycerides of saturated C₉₋₁₈ fatty acids. The hard fat base can comprise hard fats obtained by esterification of fatty acids of vegetable origin with glycerol, a macrogol ether containing 20 to 24 oxyethylene groups in the polyoxyethylene chain, e.g., polyoxyl-20-cetostearyl ether, and glycerides, e.g., glyceryl ricinoleate. Other suitable suppository bases include cocoa butter, lauric oil, beef tallow, hard fat, and any combination of any of the foregoing. The drug load of the suppository is, for example, between 30 and 60%, for example 35 to 50% or 37% to 50%. According to one embodiment, the drug load ranges from about 37 to about 46%. According to some embodiments, the drug load ranges from about 39 to about 45%. According to some examples, the drug load ranges from about 41 to about 43%. For example, the suppository can contain about 1000 mg compound of Formula I dispersed in about 1300 to about 1500 mg of a suppository base (preferably hard fat). In some examples, the total weight of the suppository ranges from about 500 to 5000 mg, from 1000 to 4000 mg, from 1500 to 3500 mg, from 2250 to about 2700 mg or from about 2250 to about 2500 mg. The suppository is preferably smooth torpedo-shaped. The melting point of the suppository is generally sufficient to melt in the patient's body, and is typically no more than about 37° C.

In certain examples and embodiments, pharmaceutical compositions are formulated in any conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are optionally used as suitable. Pharmaceutical compositions comprising nadolol are manufactured in a conventional manner, such as, by way of example only, by means of conventional mixing, dissolving, granulating, dragee-making, levigating, emulsifying, encapsulating, entrapping or compression processes.

Pharmaceutical compositions include at least one pharmaceutically acceptable carrier, diluent or excipient and nadolol. The active ingredient is in free-acid or free-base form, or in a pharmaceutically acceptable salt form. In addition, the pharmaceutical compositions described herein include the use of crystalline forms (also known as polymorphs), of nadolol. Additionally, nadolol encompasses unsolvated as well as solvated forms with pharmaceutically acceptable solvents such as water or ethanol. The solvated forms of nadolol are also disclosed herein. In addition, the pharmaceutical compositions optionally include other medicinal or pharmaceutical agents, carriers, adjuvants, such as preserving, stabilizing, wetting or emulsifying agents, solution promoters, salts for regulating the osmotic pressure, buffers, and/or other therapeutically valuable substances. Methods for the preparation of compositions comprising the compounds described herein include formulating the compounds with one or more inert, pharmaceutically acceptable excipients or carriers to form a solid, semi-solid or liquid. Solid compositions include powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein. Semi-solid compositions include gels, suspensions and creams. The form of the pharmaceutical compositions described herein include liquid solutions or suspensions, solid forms suitable for solution or suspension in a liquid prior to use, or as emulsions. These compositions also optionally contain minor amounts of nontoxic, auxiliary substances, such as wetting or emulsifying agents, pH buffering agents, and so forth.

In some examples and embodiments, pharmaceutical composition comprising nadolol illustratively takes the form of a liquid where the agents are present in solution, in suspension or both. Typically when the composition is administered as a solution or suspension a first portion of the agent is present in solution and a second portion of the agent is present in particulate form, in suspension in a liquid matrix. In some embodiments, a liquid composition includes a gel formulation. In some embodiments, the liquid composition is aqueous.

In certain embodiments, useful aqueous suspensions contain one or more polymers as suspending agents. Useful polymers include water-soluble polymers such as cellulosic polymers, e.g., hydroxypropyl methylcellulose, and water-insoluble polymers such as cross-linked carboxyl-containing polymers. Certain pharmaceutical compositions described herein comprise a mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Useful pharmaceutical compositions also optionally include solubilizing agents to aid in the solubility of nadolol. The term "solubilizing agent" generally includes agents that result in formation of a micellar solution or a true solution of the agent. Certain acceptable nonionic surfactants, for example polysorbate 80, are useful as solubilizing agents, as can ophthalmically acceptable glycols, polyglycols, e.g., polyethylene glycol 400, and glycol ethers.

Furthermore, useful pharmaceutical compositions optionally include one or more pH adjusting agents or buffering agents, including acids such as acetic, boric, citric, lactic, phosphoric and hydrochloric acids, bases such as sodium hydroxide, sodium phosphate, sodium borate, sodium citrate, sodium acetate, sodium lactate and tris-hydroxymethylaminomethane, and buffers such as citrate/dextrose, sodium bicarbonate and ammonium chloride. Such acids, bases and buffers are included in an amount required to maintain pH of the composition in an acceptable range.

Additionally, useful compositions also optionally include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions, suitable salts include sodium chloride, potassium chloride, sodium thiosulfate, sodium bisulfite and ammonium sulfate.

Other useful pharmaceutical compositions optionally include one or more preservatives to inhibit microbial activity. Suitable preservatives include mercury-containing substances such as merfen and thiomersal, stabilized chlorine dioxide, and quaternary ammonium compounds such as benzalkonium chloride, cetyltrimethylammonium bromide and cetylpyridinium chloride.

Still other useful compositions include one or more surfactants to enhance physical stability or for other purposes. Suitable nonionic surfactants include polyoxyethylene fatty acid glycerides and vegetable oils, e.g., polyoxyethylene (60) hydrogenated castor oil, and polyoxyethylene alkylethers and alkylphenyl ethers, e.g., octoxynol 10, octoxynol 40.

Still other useful compositions include one or more antioxidants to enhance chemical stability where required. Suitable antioxidants include, by way of example only, ascorbic acid and sodium metabisulfite.

In certain embodiments, aqueous suspension compositions are packaged in single-dose non-reclosable containers. Alternatively, multiple-dose reclosable containers are used, in which case it is typical to include a preservative in the composition.

In alternative embodiments, other delivery systems for hydrophobic pharmaceutical compounds are employed. Liposomes and emulsions are examples of delivery vehicles or carriers useful herein. In certain embodiments, organic solvents such as *N*-methylpyrrolidone are also employed. In additional embodiments, the compounds described herein are delivered using a sustained-release system, such as semipermeable matrices of solid hydrophobic polymers containing the therapeutic agent. Various sustained-release materials are useful herein. In some embodiments, sustained-release capsules release the compounds for a few weeks up to over 100 days. Depending on the chemical nature and the biological stability of the therapeutic reagent, additional strategies for protein stabilization are employed.

In certain embodiments, the formulations described herein comprise one or more antioxidants, metal chelating agents, thiol containing compounds and/or other general stabilizing agents. Examples of such stabilizing agents: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc, or (n) combinations thereof.

Preferred embodiments of the present invention are described herein.

### Modulation of gene and signaling pathways embodiments

In some examples, the nadolol has one or more effects in a cell including anti-inflammatory, immunosuppressive and other immunodulatory effects. In eliciting these effects, nadolol modulates one or more genes and signal transduction pathways or networks in the cell or body of the subject.

In some examples, nadolol modulates a signal transduction pathway including a TREM1 signaling pathway, an antigen presentation pathways, a TGF-β pathway, a T-cell or B-cell pathway, a toll-like receptor pathway, a complement system pathway, a leukocyte extravasation pathway, a T helper (Tₕ) cell differentiation pathway, a graft-versus-host disease pathway, an autoimmune thyroid disease pathway, a macrophage pathway, a fibroblast or endothelial cell pathway (e.g. a pathway involved in rheumatoid arthritis), an IL-12 signaling and/or production pathway, a cytokine-mediated communication pathway (e.g. between immune cells), a dendritic cell maturation pathway, a systemic lupus erythematosus pathway, a B-cell development pathway, and an airway inflammation (asthma) pathway.

In some examples, nadolol modulates one or more pathway, including canonical pathways that are relevant to inflammatory bowel disease and/or autoimmune disease. Examples of pathways which are modulated by compounds of Formula I include pathways for: crosstalk between dendritic cells and natural killer cells, cytokine mediated communication between immune cells, communication between innate and adaptive immune cells, differential regulation of cytokine production in intestinal epithelial cells by IL-1A and IL-17F, altered T-cell and B-cell signaling in rheumatoid arthritis, leukocyte extravasation signaling, complement system, LPS/IL-1 mediated inhibition of RXR function, differential regulation of cytokine production in macrophages and T helper cells by IL-17A and IL-17F, and/or dendritic cell maturation.

In some examples, nadolol modulates the activity of the TREM1 pathway. TREM1 belongs to the Immunoglobulin (Ig) family of cell surface receptors and is selectively expressed on blood neutrophils, monocytes and macrophages. TREM1 lacks known signaling motifs in the cytoplasmic domain and thus activation by TREM1 is believed to be mediated by a transmembrane adaptor molecule DNAX-activating protein 12 (DAP12), leading to proinflammatory immune responses. The natural ligand for TREM1 is unknown. TREM1 activation triggers the Janus kinase 2 (JAK2), protein kinase B (PKB/AKT) and extracellular signal related kinase (ERK1/2) pathways leading to the phosphorylation of signal transducers of activation of transcription (STAT3,5) and NF kappa B (NF-kB). These transcription factors upregulate the expression of genes involved in the inflammatory response. Stimulation of TREM1 by its ligand or toll like receptor (TLR) by lipopolysaccharide (LPS) can lead to an association of TREM1 and TLR. This association leads to the activation of interleukin-1 receptor-associated kinase 1 (IRAKI), which in turn triggers NF-kB and the proinflammatory response. Engagement and activation of TREM1 triggers expression and secretion of chemokines and cytokines like monocyte chemotactic protein 1(MCP-1), macrophage inflammatory protein-1alpha (MIP-1a), interleukins (IL-6,-8) and tumor necrosis factor (TNF). Many of these effects are potentiated by LPS. Cytokines like TNF and granulocyte macrophage colony stimulating factor (GM-CSF) in turn upregulate the expression of TREM1 in an autocrine fashion. The synergy between TLR and TREM1 leads to neutrophil degranulation, phagocytosis and the respiratory burst in addition to the production of proinflammatory cytokines. TREM1 activation also results in the upregulation of cell surface proteins such as CD11, CD29 and CD40, CD83 that are involved in cell adhesion and costimulation respectively, as well as phospholipase gamma (PLCg) mediated Ca²⁺ release. Thus TREM1 activation is involved in diverse aspects of innate and adaptive immune response. In addition to TLRs, TREM1 also synergizes with a second major class of pattern recognition receptors -the NACHT-LRR receptors (NLR), which recognize intracellular microorganisms. The TREM1/NLR synergism results in the production of proinflammatory cytokines like TNF, IL-1b, IL-6 and IL-18- the latter three via a caspase-1 dependent pathway. Thus TREM1 acts to amplify signals from both major pathways of pattern recognition - extracellular TLR receptors and the intracellular NLR proteins. By interacting with one or more components of the TREM1 signaling pathway, nadolol modulates the activity of this pathway.

In some examples, nadolol modulates the activity of an antigen presentation pathway. These pathways mediate a process in the body's immune system by which macrophages, dendritic cells and other cell types capture antigens and then enable their recognition by T-cells. Antigen presentation pathways include the endogenous pathway, which is used to present cellular peptide fragments on the cell surface on MHC class I molecules, and the exogenous pathway, which is utilized by specialized antigen presenting cells to present peptides derived from proteins that the cell has endocytosed, and which peptides are presented on MHC class II molecules. By interacting with one or more components of an antigen presentation pathway(s), nadolol modulates the activity of these pathways.

In some examples, nadolol modulates the activity of a TGF-β pathway. The TGF-β superfamily of ligands include: bone morphogenetic proteins (BMPs), growth and differentiation factors (GDFs), anti-Müllerian hormone (AMH), Activin, Nodal and TGFβ's. Signaling is believed to begin with the binding of a TGF-β superfamily ligand to a TGF-β type II receptor. The type II receptor is a serine/threonine receptor kinase, which catalyzes the phosphorylation of the Type I receptor. Each class of ligand is believed to bind to a specific type II receptor. Mammals have seven known type I receptors, five type II receptors. Three activins are known, and include Activin A, Activin B and Activin AB. Activins are involved in embryogenesis and osteogenesis, and regulate hormones including, but not limited to pituitary, gonadal and hypothalamic hormones, as well as insulin. Activins also act as nerve cell survival factors. BMPs bind to the Bone morphogenetic protein receptor type-2 (BMPR2), and are involved in a multitude of cellular functions including osteogenesis, cell differentiation, anterior/posterior axis specification, growth, and homeostasis. Nodal is involved in cell differentiation, and is believed to be involved in mesoderm formation and subsequent organization of left-right axial structures in early embryonic development. The TGF-β family further includes ligands such as TGFβ1, TGFβ2, and TGFβ3. By interacting with one or more components of a TGF-β pathway, nadolol modulates the activity of this pathway.

In some examples, nadolol modulates the activity of a T-cell or B-cell pathway. Such pathways are, for example, involved in rheumatoid arthritis (RA). RA is a form of autoimmunity, the causes of which are still incompletely known, but is believed to involve abnormal B cell-T cell interaction. Inflammation can be driven either by B cell or T cell products, stimulating release of TNF and other cytokines. By interacting with one or more components of a T-cell or B-cell pathway, nadolol modulates the activity of this pathway.

In some examples, nadolol modulates the activity of a Toll-like receptor pathway. Toll-like receptors (TLRs) recognize distinct pathogen-associated molecular patterns and play a critical role in innate immune responses. They participate in the first line of defense against invading pathogens and play a significant role in inflammation, immune cell regulation, survival, and proliferation. A number of the TLR family have been identified, of which TLR1, TLR2, TLR4, TLR5, and TLR6 are located on the cell surface and TLR3, TLR7, TLR8, and TLR9 are localized to the endosomal/lysosomal compartment. The activation of the TLR signaling pathway originates from the cytoplasmic Toll/IL-1 receptor (TIR) domain that associates with a TIR domain-containing adaptor, MyD88. Upon stimulation with ligands, MyD88 recruits IL-1 receptor-associated kinase-4 (IRAK-4) to TLRs through interaction of the death domains of both molecules. IRAK-1 activated by phosphorylation then associates with TRAF6, finally leading to activation of MAP kinases (JNK, p38 MAPK) and NF-κB. Tollip and IRAK-M interact with IRAK-1 and negatively regulate the TLR-mediated signaling pathways. Additional modes of regulation for these pathways include TRIF-dependent induction of TRAF6 signaling by RIP1 and negative regulation of TIRAP mediated downstream signaling by ST2L, TRIAD3A, and SOCS1. MyD88-independent pathways induce activation of IRF3 and expression of interferon-β. TIR-domain containing adaptors such as TIRAP, TRIF, and TRAM regulate TLR-mediated signaling pathways by providing specificity for individual TLR signaling cascades. By interacting with one or more components of a Toll-like receptor pathway, nadolol modulates the activity of this pathway.

In some examples, nadolol modulates the activity of a complement system pathway. The complement system has four major function, including lysis of infectious organisms, activation of inflammation, opsonization and immune clearance. Complement pathways include the classical complement pathway, the alternative complement pathway, and the mannose-binding lectin pathway. The classic complement pathway is triggered when antibody-antigen complex interact with C1-complex, which consists of C1q, two molecules of C1r, and two molecules of Cls. The C1-complex cleaves C2 and C4, which then form C3 convertase (C4b2a). C3 is then cleaved by the C3 convertase, and forms C5 convertase in association with C4b and C2a. The generation of C5 convertase is the end of the classical pathway. The lectin pathway is similar to the classical pathway, and is stimulated when the mannose-binding lectin (MBL) binds to mannose residues on the pathogen surface. The MBL-associated serine proteases, MASP-1, and MASP-2, are activated and cleave C4 and C2, which then form the C3 convertase as in the classical pathway. The alternative complement pathway begins with the activation of C3 and requires factor B and factor D. All three pathways merge at C3, which is then converted into C3a and C3b. The further formed C5 convertase from C3b cleaves C5 into C5a and C5b. C5b with C6, C7, C8, and C9 complex to form the membrane attack complex (MAC), which is inserted into the cell membrane, forms a hole in the membrane, and initiates cells lysis. At least 12 proteins are known to be involved in regulation of the complement system, including Factor H, Factor I, C1 inhibitor, and CD59. Factor H removes Bb from the alternative pathway C3 convertase, breaking the positive feedback loop. Factor I inactivates C3b. C1 inhibitor binds to sites on activated C1r and C1s shutting down their proteolytic activity. CD59, also known as protectin, inhibits C9 polymerization during the formation of the membrane attack complex. By interacting with one or more components of a complement system pathway, a nadolol modulates the activity of this pathway.

In some examples, a compound of Formula I modulates the activity of a leukocyte extravasation pathway. This pathway stimulates the movement of leukocytes out of the circulatory system, towards the site of tissue damage or infection. Upon recognition of and activation by pathogens, resident macrophages in the affected tissue release cytokines such as IL-1, TNFα and chemokines. IL-1 and TNFα cause the endothelial cells of blood vessels near the site of infection to express cellular adhesion molecules, including selectins and integrins. Circulating leukocytes are localized towards the site of injury or infection due to the presence of chemokines. By interacting with one or more components of a leukocyte extravasation pathway, nadolol modulates the activity of this pathway.

In some examples, nadolol modulates the activity of a T helper cell (Tₕ) differentiation pathway. Tₕ cell's progenitors differentiate into effector Tₕ cells, memory Tₕ cells, and regulatory Tₕ cells. Once two-signal activation is complete, Tₕ proliferates by releasing a T-cell growth factor called interleukin 2 (IL-2), which acts in an autocrine fashion. Activated T-cells also produce the alpha sub-unit of the IL-2 receptor (CD25 or IL-2R), enabling a fully functional receptor that can bind with IL-2, which in turn activates the T cell's proliferation pathways. The autocrine or paracrine secretion of IL-2 can bind that same Tₕ cell or neighboring Tₕ's via the IL-2R thus driving proliferation and clonal expansion. The Tₕ cells receiving both signals of activation will then become Tₕ0 cells (T helper 0) cell that secrete IL-2, IL-4 and interferon gamma (IFN-y). The Tₕ0 cells will then differentiate into Tₕ1 or Tₕ2 cells depending on cytokine environment. IFN-y drives Tₕ1 cell production while IL-10 and IL-4 inhibit Tₕ1 cell production. Conversely, IL-4 drives Tₕ2 cell production and IFN-y inhibits Tₕ2 cells. Such cytokines are pleiotropic and carry out many other functions of the immune response. By interacting with one or more components of a Tₕ differentiation pathway, nadolol modulates the activity of this pathway.

In some examples, nadolol modulates the activity of a graft-versus-host disease (GVHD) signaling pathway. This pathway is involved in GVHD, which is a lethal complication of allogeneic hematopoietic stem cell transplantation (HSCT) where immunocompetent donor T-cells attack the genetically disparate host cells. GVHD pathophysiology involves a three-step process, where the first step is the development of an inflammatory milieu resulting from damage in the host tissues induced by the preparative chemotherapy or radiotherapy regimen. Damaged tissues secrete inflammatory cytokines, including interleukin 1 (IL-1), and tumor necrosis factor (TNF-alpha). During the second step, antigen-presenting cells (APCs) trigger the activation of donor-derived T cells, which induce further T-cell expansion, induce cytotoxic T lymphocytes (CTL) and natural killer (NK) cells responses and prime additional mononuclear phagocytes to produce TNF-alpha and IL-1. Also, nitric oxide (NO) is produced by activated macrophages, and it may contribute to the tissue damage observed subsequently. During the third step, the effector phase, activated CTL and NK cells mediate cytotoxicity against target host cells through Fas-Fas ligand interactions and perforin-granzyme B. By interacting with one or more components of the GVHD pathway, nadolol modulates the activity of this pathway.

In some examples, nadolol modulates the activity of a pathway which controls or determines the function of macrophages, fibroblast and endothelial cells. Such pathways are important, for example, in disorders such as rheumatoid arthritis (RA). The pathology of RA is characterized by the infiltration of several inflammatory cells into both the pannus and the joint fluid, and by subsequent tissue destruction. Chemokines, as well as other inflammatory mediators play key roles in the pathogenesis of RA, and the coordinated production of chemokines and pro-inflammatory cytokines is important in the orchestration of the inflammatory responses observed in patients with RA. Monocytes that are attracted to the RA joint differentiate into macrophages and become activated. These macrophages play a pivotal role in RA because they are numerous in the inflamed synovial membrane and at the cartilage-pannus junction. They activate MHC Class-II (Major Histocompatibility Complex Class-II) molecules, and secrete proinflammatory or regulatory cytokines and growth factors like IL-1, IL-2, IL-6, IL-10, IL-13, IL-15, IL-17, IL-18, TNF-Alpha (Tumor Necrosis Factor), GMCSF (Granulocyte-Macrophage Colony-Stimulating Factor), chemokines and chemoattractants (e.g. IL-8, MIP1 (Macrophage Inflammatory Protein-1) and MCP1 (Monocyte Chemoattractant Protein), metalloproteinases and neopterin. TNF regulates IL-1β expression, which is important for the induction of prostanoid and MMP (Matrix Metalloproteinases) production by synovial fibroblasts and chondrocytes. Cellular interactions mediated by TNF and IL-1 cytokines that are mainly produced by activated macrophages are prominent factors leading to cartilage damage in RA. TNF increases the expression of adhesion molecules on endothelial cells, which recruit more cells to the joint. MCP1 and IL-8 are also secreted by macrophages and attract more cells into the joint. IL-1 and TNF induce synovial fibroblasts to express IL-6, chemokines, GMCSF and MMPs, which contribute to cartilage and bone destruction. TNF contributes to osteoclast activation and differentiation. In addition, IL-1 mediates cartilage degradation directly by inducing the expression of MMPs by chondrocytes. However, these cells of the innate immune system possess broad proinflammatory, destructive and remodeling capacities, and considerably contribute to inflammation and joint destruction both in the acute and chronic phases of RA. In addition, these chemokines produced by RA synovial stromal cells also stimulate monocyte migration. Other cytokines such as Ifn-Gamma (Interferon-Gamma) induced chemokines also contribute to the documented morphologic and clinical features of RA. The etiology of RA also involves abnormal presentation of self antigen(s) by APCs (Antigen Presenting Cells) and activation of autoreactive T-cells. T lymphocytes play a central role in the disease process. The rheumatoid synovial membrane is rich in MHC Class-II, APCs, and CD4+ T-Cells. APCs require signals from activated T-cells for their differentiation and maturation, this subsequently enables APCs to activate newly arrived T-cells in a specific or unspecific manner in the local inflammation. Activated T-cells promote the disease progression by inducing the secretion of pro-inflammatory cytokines (TNF-Alpha) from macrophages and synovial cells in a contact-dependent manner. Several costimulatory molecules are involved during APC-T-Cell interactions, including CD28/CD80-86 and CD40-CD40L. Some of these molecules are critical in initiation of the immune response (CD28/CD80-86), while CD40-CD40L is required for the amplification of the inflammatory response. By interacting with one or more components of a macrophage, fibroblast, and endothelial cell pathway, nadolol modulates the activity of this pathway.

In some examples nadolol modulates the activity of IL-12 signaling and production in macrophages pathway. Interleukin-12 is a heterodimeric cytokine, mainly produced by macrophages. IL-12 plays an important role in the activities of natural killer cells and T lymphocytes. IL-12 mediates enhancement of the cytotoxic activity of NK cells and CD8+ cytotoxic T lymphocytes. IL-2 is also linked to signal transduction in NK cells. IL-2 stimulates the expression of two IL-12 receptors, IL-12R-β1 and IL-12R-β2, maintaining the expression of a critical protein involved in IL-12 signaling in NK cells. Enhanced functional response is demonstrated by IFN-y production and killing of target cells. IL-12 also has anti-angiogenic activity, which it controls by increasing production of interferon gamma, which in turn, increases the production of a chemokine called inducible protein-10 (IP-10 or CXCL10) which then mediates the anti-angiogenic effect. Abnormal signaling by IL-12 is known to be involved in diseases which include autoimmune diseases, psoriasis and inflammatory bowel disease. By interacting with a pathway involving IL-12, nadolol modulates the activity of this pathway.

In some examples, nadolol modulates a pathway which regulates the communication between immune cell pathways, for example by modulating the function of cytokines. Cytokines are small cell-signaling protein molecules that are secreted by numerous cells and are a category of signaling molecules used extensively in intercellular communication. Nadolol can modulate the activity of cytokines by interacting with pathways which control their function.

In some examples, nadolol modulates the activity of a dendritic cell maturation pathway. DCs are capable of evolving from immature, antigen-capturing cells to mature, antigen-presenting, T cell-priming cells, converting antigens into immunogens and expressing molecules such as cytokines, chemokines, costimulatory molecules and proteases to initiate an immune response. The types of T cell-mediated immune responses (tolerance vs. immunity, Th1 vs. Th2) induced can vary, however, depending on the specific DC lineage (myeloid DC Is or lymphoid DC2s) and maturation stage in addition to the activation signals received from the surrounding microenvironment. The ability of DCs to regulate immunity is dependent on DC maturation. A variety of factors can induce maturation following antigen uptake and processing within DCs, including: whole bacteria or bacterial-derived antigens (e.g. lipopolysaccharide, LPS), inflammatory cytokines, ligation of select cell surface receptors (*e*.*g*. CD40) and viral products (*e*.*g*. double-stranded RNA). During their conversion from immature to mature cells, DCs undergo a number of phenotypical and functional changes. The process of DC maturation, in general, involves a redistribution of major histocompatibility complex (MHC) molecules from intracellular endocytic compartments to the DC surface, down-regulation of antigen internalization, an increase in the surface expression of costimulatory molecules, morphological changes (e.g. formation of dendrites), cytoskeleton re-organization, secretion of chemokines, cytokines and proteases, and surface expression of adhesion molecules and chemokine receptors. By interacting with a dendritic cell maturation pathway, nadolol modulates the activity of this pathway.

In some examples, nadolol modulates the activity of a pathway which mediates systemic lupus erythematosus (SLE). In SLE, the body's immune system produces antibodies against itself, particularly against proteins in the cell nucleus. SLE is triggered by environmental factors that are unknown. During an immune reaction to a foreign stimulus, such as bacteria, virus, or allergen, immune cells that would normally be deactivated due to their affinity for self tissues can be abnormally activated by signaling sequences of antigen-presenting cells. Thus triggers may include viruses, bacteria, allergens (both IgE and hypersensitivity), and can be aggravated by environmental stimulants such as ultraviolet light and certain drug reactions. These stimuli begin a reaction that leads to destruction of other cells in the body and exposure of their DNA, histones, and other proteins, particularly parts of the cell nucleus. The body's sensitized B-lymphocyte cells will now produce antibodies against these nuclear-related proteins. These antibodies clump into antibody-protein complexes which stick to surfaces and damage blood vessels in critical areas of the body, such as the glomeruli of the kidney, these antibody attacks are the cause of SLE. SLE is a chronic inflammatory disease believed to be a type III hypersensitivity response with potential type II involvement. High mobility group box 1 (HMGB 1) is a nuclear protein participating in chromatin architecture and transcriptional regulation which was found in the sera of patients and mice with systemic lupus erythematosus. HMGB1 may contribute to the pathogenesis of chronic inflammatory and autoimmune diseases due to its proinflammatory and immunostimulatory properties. By interacting with a pathway involved in SLE, nadolol modulates the activity of this pathway.

In some examples, nadolol selectively decreases activity of one or more genes. Such genes include BSN (Zinc Finger Protein 231), CD3E (T-Cell Antigen Receptor Complex, Epsilon Subunit Of T3), F10 (Coagulation Factor X), IFNAR1 (Interferon, Alpha, Beta, And Omega, Receptor 1), MMP9 (Matrix Metalloproteinase 9), IL-2 (Interleukin 2), IL-25 (Interleukin 25), IL2RA (Interleukin 2 Receptor, Alpha), TLR2 (Toll-Like Receptor 2), IL-10 (Interleukin 10), and IGF1 (Insulin-Like Growth Factor I).

In some examples, nadolol selectively decreases activity of one or more genes. Such genes include genes known to be associated with IBD, for example CXCL10, MMP9, IGF1, and IL10. In some embodiments nadolol down-regulates a gene associate with UC, for example CXCL10 or MMP9. In some embodiments nadolol down-regulates a gene associated with Crohn's disease, for example IGF1 or IL10.

### Methods of Treatment

Provided herein is nadolol for use in treating an inflammatory bowel disorder in a human in need thereof wherein the nadolol is formulated for release in the small intestine or the large intestine of the human.

In some embodiments, the administering step of the compound for use results in an improvement the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject.

In some embodiments, the administering step of the compound for use occurs prior to, concurrent with, or after administration of an additional treatment regimen or therapeutic agent to the subject. In some embodiments, the additional therapeutic agent is a steroid, more particularly a corticosteroid, or other anti-inflammatory drug. In some embodiments, the additional treatment regimen comprises traditional or laparoscopic surgery.

In some embodiments, the administering step of the compound for use occurs prior to, concurrent with, or after administration of an additional treatment to said subject, wherein the additional treatment is a laxative or anti-constipation agent. In some embodiments, the additional therapeutic agent is an antacid or other acidic reducer.

Nadolol is administered, for example, via pH-dependent release, via microbially-triggered delivery, as a conjugate, via time-controlled delivery, osmotically-regulated delivery, pressure-controlled delivery, a multi matrix system delivery, bioadhesion delivery, or multiparticulate delivery.

In some examples, the compound is released preferentially in the small or large intestine, colon, rectum, stomach, anus, or esophagus. In some examples, at least 60%, 70%, 80%, or 90% of the administered dose of the compound is released.

In some examples, an effective therapeutic amount of a composition comprising nadolol is applied to a gastrointestinal tissue and reduces the occurrence of a symptom such as inflammation, swelling, irritation, open sores, bleeding, ulcers, fistulas, abdominal pain, or produces an improvement in defecation in the subject. Exemplary target sites of the compound include the esophagus, stomach, large and small intestine, sigmoid colon, and rectum in a subject.

In some examples, provided herein is a therapeutically effective amount of nadolol for use in a method of treating Crohn's disease in a human in need thereof wherein the nadolol is formulated for release in the small intestine or the large intestine of the human. Crohn's disease is a type of inflammatory bowel disease which can attack any part of the digestive tract. It typically manifests in the gastrointestinal tract and can be categorized by the specific tract region affected. A disease of the ileum and the large intestine, ileocolic Crohn's accounts for fifty percent of cases. Crohn's ileitis, manifested in the ileum only, accounts for thirty percent of cases, while Crohn's colitis, of the large intestine, accounts for the remaining twenty percent of cases. Gastroduodenal Crohn's disease causes inflammation in the stomach and the duodenum. Jejunoileitis causes spotty patches of inflammation in the jejunum. There are three categories of disease presentation in Crohn's disease: stricturing, penetrating, and inflammatory. Stricturing disease causes narrowing of the bowel that may lead to bowel obstruction or changes in the caliber of the feces. Penetrating disease creates abnormal passageways (fistulae) between the bowel and other structures, such as the skin. Inflammatory disease (or nonstricturing, nonpenetrating disease) causes inflammation without causing strictures or fistulae.

Treatment of Crohn's includes uses of antibiotics and aminosalicylate anti-inflammatory drugs and corticosteroids. When symptoms are in remission, treatment enters maintenance, with a goal of avoiding the recurrence of symptoms. Alternatives treatments include aminosalicylates alone and many require immunosuppressive drugs. It has been also suggested that antibiotics change the enteric flora, and their continuous use may pose the risk of overgrowth with pathogens such as *Clostridium difficile.* Medications used to treat the symptoms of Crohn's disease include 5-aminosalicylic acid formulations, prednisone, immunomodulators such as azathioprine, mercaptopurine, methotrexate, infliximab, adalimumab, certolizumab and natalizumab. Hydrocortisone is also used in severe attacks of Crohn's disease. Surgery is used when partial or a full blockage of the intestine occurs. Surgery may also be required for complications such as obstructions, fistulas and/or abscesses, or if the disease does not respond to drugs. Any of these therapeutic agents and methods can be used in conjunction with compositions comprising nadolol.

In some embodiments, provided herein is a composition comprising a therapeutically effective amount of nadolol for use in treating UC in a human in need thereof wherein the nadolol is formulated for release in the small intestine or the large intestine of the human. This disease is a form of inflammatory bowel disease (IBD) of the colon (large intestine), which includes characteristic ulcers, or open sores. UC can be treated with a number of medications including aminosalicylates such as sulfasalazine, corticosteroids such as prednisone, immunosuppressive medications such as azathioprine, and biological agents such as infliximab. UC can generally be cured by surgical removal of the large intestine, also known as a colectomy. This procedure is necessary in the event of exsanguinating hemorrhage, frank perforation or documented or strongly suspected carcinoma. Any of these therapeutic agents can be used in conjunction with compositions comprising nadolol.

In some examples, provided herein is a composition comprising a therapeutically effective amount of nadolol for use in a method of treating other gastrointestinal diseases in a human in need thereof wherein the nadolol is formulated for release in the small intestine or the large intestine of the human. Such gastrointestinal diseases include achalasia, Barrett's oesophagus, colorectal cancer, gastric cancer, oesophageal cancer, celiac disease, colitis, diverticulosis, diverticulitis, gastritis, irritable bowel syndrome, microscopic colitis, collagenous colitis, lymphocytic colitis, pancreatitis and reflux oesophagitis.

In some examples, provided herein is a composition comprising a therapeutically effective amount of nadolol for use in a method of treating achalasia in a human in need thereof wherein the nadolol is formulated for release in the small intestine or the large intestine of the human. Achalasia is a disorder of the esophagus. It is an esophageal motility disorder involving the smooth muscle layer of the esophagus and the lower esophageal sphincter (LES). It is characterized by incomplete LES relaxation, increased LES tone, and lack of peristalsis of the esophagus (inability of smooth muscle to move food down the esophagus) in the absence of other explanations like cancer or fibrosis. The cause of most cases of achalasia is unknown. LES pressure and relaxation are regulated by excitatory (eg, acetylcholine, substance P) and inhibitory (eg, nitric oxide, vasoactive intestinal peptide) neurotransmitters. Persons with achalasia lack nonadrenergic, noncholinergic, inhibitory ganglion cells, causing an imbalance in excitatory and inhibitory neurotransmission. The result is a hypertensive nonrelaxed esophageal sphincter. Achalasia is characterized by difficulty swallowing, regurgitation, and sometimes chest pain. Diagnosis is reached with esophageal manometry and barium swallow radiographic studies. Various treatments are available. Certain medications or botulinum toxin may be used in some cases, but more permanent relief is brought by esophageal dilatation and surgical cleaving of the muscle (Heller myotomy). Any of these therapeutic agents and methods can be used in conjunction with compositions comprising nadolol.

In some examples, provided herein is a composition comprising a therapeutically effective amount of nadolol for use in a method of treating Barrett's oesophagus disease in a human in need thereof wherein the nadolol is formulated for release in the small intestine or the large intestine of the human. This disease is marked by the presence of columnar epithelia in the lower esophagus, replacing the normal squamous cell epithelium. The main cause of Barrett's esophagus is thought to be an adaptation to chronic acid exposure from reflux esophagitis. Barrett's esophagus is found in a fraction of patients who seek medical care for heartburn (gastroesophageal reflux disease, GERD), although a large subgroup of patients with Barrett's esophagus do not have symptoms. Treatment options for high-grade dysplasia include surgical removal of the esophagus (esophagectomy) or endoscopic treatments such as endoscopic mucosal resection or ablation (destruction). Any of these therapeutic options can be used in conjunction with compositions comprising nadolol.

In some examples, provided herein is a composition comprising a therapeutically effective amount of nadolol for use in method of treating gastroesophageal reflux disease (GERD) in a human in need thereof wherein the nadolol is formulated for release in the small intestine or the large intestine of the human. The principal cause of gastroesophageal reflux disease is chronic inflammation. In this disease, acidic stomach, bile, small intestine and pancreatic contents cause damage to the cells of the lower esophagus. Bile acids may induce intestinal differentiation in gastroesophageal junction cells through inhibition of the epidermal growth factor receptor (EGFR) receptor which results in inhibition of Akt, upregulation of the p50 subunit of NF-kB (NFKB1) and ultimately activation of the promotor of the homeobox gene CDX2. Treatment options for GERD include lifestyle modifications (dietary changes or alterations of sleeping position), medications (proton pump inhibitors, including but not limited to omeprazole, esomeprazole, pantoprazole, lansoprazole, and rabeprazole, gastric H2 receptor blockers such as ranitidine, famotidine and cimetidine, antacids, alginic acid, prokinetics such as cisapride, or metoclopriamide, sucralfate, mosapride citrate, GABA_{B} receptor agonists such as baclofen), and surgery (Nissen fundoplication or vagotomy). Any of the named therapeutic agents and methods can be used in conjunction with compositions comprising nadolol.

In some examples, provided herein is a composition comprising a therapeutically effective amount of nadolol for use in a method of treating colorectal cancer (CRC) in a human in need thereof wherein the nadolol is formulated for release in the small intestine or the large intestine of the human. CRC is a disease originating from the epithelial cells lining the colon or rectum of the gastrointestinal tract, most frequently as a result of mutations in the Wnt signaling pathway that artificially increase signaling activity. The most commonly mutated gene in all colorectal cancer is the APC gene, which produces the APC protein. The APC protein is a "brake" on the accumulation of β-catenin protein, without APC, β-catenin accumulates to high levels and translocates into the nucleus, binds to DNA, and activates the transcription of genes that are normally important for stem cell renewal and differentiation but when inappropriately expressed at high levels can cause cancer. The symptoms and signs of colorectal cancer depend on the location of tumor in the bowel. The classic warning signs of CRC include: worsening constipation, blood in the stool, weight loss, fever, loss of appetite, and nausea or vomiting. Any of the named therapeutic agents and methods can be used in conjunction with compositions comprising a nadolol.

In some examples, provided herein is a composition comprising a therapeutically effective amount of nadolol for use in a method of treating gastric cancer in a human in need thereof wherein the nadolol is formulated for release in the small intestine or the large intestine of the human. Gastric cancer is characterized by a malignancy of rising from any part of the stomach. Stomach cancer is often asymptomatic or causes only nonspecific symptoms in its early stages such as abdominal discomfort, indigestion, and loss of appetite. There are two major pathways for the development of gastric cancer by *Helicobacter pylori* infection: the indirect action of *H. pylori* on gastric epithelial cells through inflammation, and the direct action of the bacteria on epithelial cells through the induction of protein modulation and gene mutation. Both pathways work together to promote gastric carcinogenesis. The onset of autoimmune atrophic gastritis, intestinal metaplasia and various genetic factors are associated with increased risk for development of gastric cancer. Treatment for stomach cancer may include surgery, chemotherapy, and/or radiation therapy. Any of the named therapeutic agents and methods can be used in conjunction with compositions comprising nadolol.

In some examples, provided herein is a composition comprising a therapeutically effective amount of nadolol for use in a method of treating oesophageal cancer in a human in need thereof wherein the nadolol is formulated for release in the small intestine or the large intestine of the human. This form of cancer is characterized by a malignancy arising from the esophagus. The most common types of esophageal cancer are adenocarcinoma, which starts in the glandular cells producing fluids such as mucus, and squamous cell carcinoma, which starts in flat cells of the esophageal lining. Chronic irritation from gastroesophageal reflux disease (GERD), Barrett's esophagus, smoking, obesity and heavy alcohol use are leading risk factors for the disease. Precancerous esophageal lesions and early esophageal cancer may be treated with endoscopic therapies include endoscopic mucosal resection, radiofrequency ablation or photodynamic therapy. For more advanced cancer, esophagectomy surgery to remove a portion of the esophagus is usually necessary. Any of the named therapeutic agents and methods can be used in conjunction with compositions comprising nadolol.

In some examples, provided herein is a composition comprising a therapeutically effective amount of nadolol for use in method of treating celiac disease (celiac sprue) in a human in need thereof wherein the nadolol is formulated for release in the small intestine or the large intestine of the human. Celiac disease is an autoimmune disorder of the small intestine. It is caused by a reaction to gliadin, a prolamin (gluten protein) found in wheat, and similar proteins found in the crops of the tribe *Triticeae*, which includes common grains such as barley and rye. Upon exposure to gliadin, and specifically to three peptides found in prolamins, the enzyme tissue transglutaminase modifies the protein, and the immune system cross-reacts with the small-bowel tissue, causing an inflammatory reaction. That leads to a truncating of the villi lining the small intestine. This interferes with the absorption of nutrients, because the intestinal villi are responsible for absorption. A gluten-free diet is essential to managing celiac disease. Such dietary modifications can be used in conjunction with compositions comprising nadolol.

In some embodiments, provided herein is a composition comprising a therapeutically effective amount of nadolol for use in a method of treating colitis in a human in need thereof wherein the nadolol is formulated for release in the small intestine or the large intestine of the human. Colitis is an inflammation of the colon and is often used to describe an inflammation of the large intestine (colon, caecum and rectum). Colitides may be acute and self-limited or chronic, i.e. persistent, and broadly fit into the category of digestive diseases. There are many types of colitis, usually classified by the etiology, for example: autoimmune inflammatory bowel disease are a group of chronic colitides, UC is a chronic colitis that affects the large intestine, Crohn's disease is a type of IBD which often leads to a colitis, idiopathic microscopic colitis is a colitis which is diagnosed by microscopic examination of colonic tissue, lymphocytic colitis, collagenous colitis, iatrogenic diversion colitis, chemical colitis, vascular disease ischemic colitis, infectious colitis (e.g. *Clostridium difficile* colitis), pseudomembranous colitis, enterohemorrhagic colitis (which may be caused by Shiga toxin in *Shigella dysenteriae* or Shigatoxigenic group of *Escherichia coli*, which includes serotype O157:H7 and other enterohemorrhagic *E. coli*), colitis caused by parasitic infections, like those caused by *Entamoeba histolytica*, fulminant colitis (any colitis that becomes worse rapidly). In addition to the diarrhea, fever, and anemia seen in colitis, the patient has severe abdominal pain and presents a clinical picture similar to that of septicemia, where shock is present. About half of human patients require surgery. How a given colitis is treated is dependent on its etiology, e.g. infectious colitis is usually treated with antimicrobial agents, autoimmune mediated colitis is treated with immune modulators/immune suppressants. Severe colitis can be life-threatening and may require surgery. Any such therapeutic agents and methods can be used in conjunction with compositions comprising nadolol.

In some examples, provided herein is a composition comprising a therapeutically effective amount of nadolol for use in a method of treating diverticulosis in a human in need thereof wherein the nadolol is formulated for release in the small intestine or the large intestine of the human. Diverticulosis is characterized by outpocketings of the colonic mucosa and submucosa through weaknesses of muscle layers in the colon wall. These are more common in the sigmoid colon, which is a common place for increased pressure. The clinical forms include, for example, symptomatic colonic diverticulosis. This condition is characterized by motility (that is, the onward propulsive nature of contractions) of the bowel becomes disorganized. Sometimes, spasm can develop. This results in pain in the left lower abdomen and often is accompanied by the passage of small pellet-like stools and slime, which relieves the pain. Symptoms can consist of bloating, changes in bowel movements (diarrhea or constipation), non-specific chronic discomfort in the lower left abdomen, with occasional acute episodes of sharper pain, and abdominal pain, often in the left lower abdomen and/or after meals. Another clinical manifestation is known as complicated colonic diverticulosis. This condition is characterized by the diverticula bleeding, either rapidly (causing bleeding through the rectum) or slowly (causing anaemia). The diverticula can become infected and develop abscesses, or even perforate. The exact etiology of colonic diverticulosis has yet to be fully clarified. Increasing the amount of fiber in the diet may reduce symptoms of diverticulosis and prevent complications such as diverticulitis. Fiber keeps stool soft and lowers pressure inside the colon so that bowel contents can move through easily. Therapeutic agents such as methylcellulose (Citrucel) or psyllium (Metamucil), taken one to three times a day, are also known to help. Any such therapeutic agents and methods can be used in conjunction with compositions comprising nadolol.

In some examples, provided herein is a composition comprising a therapeutic amount of nadolol for use in a method of treating diverticulitis in a human in need thereof wherein the nadolol is formulated for release in the small intestine or the large intestine of the human. Diverticulitis is caused by inflammation of diverticula. Onset of this disease can occur when diverticula become infected or/and by development of abscesses. For mild diverticulitis, treatments include liquid or low-fiber diet, resting the colon, and avoiding foods or beverages that aggravate your symptoms and antibiotics. More severe, recurring acute attacks or complications, such as peritonitis, abscess, or fistula may require surgery to remove the ruptured section is removed and a colostomy is performed. Treatment with IV antibiotics may also be necessary. Any such therapeutic agents and methods can be used in conjunction with compositions comprising nadolol.

In some examples, provided herein is a composition comprising a therapeutically effective amount of nadolol for use in a method of treating gastritis in a human in need thereof wherein the nadolol is formulated for release in the small intestine or the large intestine of the human. Gastritis is an inflammation of the lining of the stomach. Treatment includes over-the-counter antacids which are commonly used for mild gastritis. When antacids do not provide sufficient relief, medications such as cimetidine, ranitidine, nizatidine or famotidine are often prescribed. Proton pump inhibitors such as omeprazole, lansoprazole, rabeprazole, and esomeprazole may be also used to reduce acid. Cytoprotective agents such as sucralfate, bismuth subsalicylate and misoprostol are designed to help protect the tissues that line the stomach and small intestine. Colonization of the gastric mucosa with *H. pylori* results in the development of chronic gastritis in infected individuals and in a subset of patients chronic gastritis progresses to complications (i.e. ulcer disease, gastric neoplasias, some distinct extra gastric diseases). Several regimens are used to treat *H. pylori* infection. Most use a combination of two antibiotics and a proton pump inhibitor, and bismuth may also added to the regimen. The antibiotic aids in destroying the bacteria, and the acid blocker or proton pump inhibitor relieves pain and nausea, heals inflammation, and may increase the antibiotic's effectiveness. Any such therapeutic agents and methods can be used in conjunction with compositions comprising nadolol.

In some embodiments, provided herein is a composition comprising a therapeutically effective amount of nadolol for use in a method of treating irritable bowel syndrome (IBS) in a human in need thereof wherein the nadolol is formulated for release in the small intestine or the large intestine of the human. IBS is characterized by chronic abdominal pain, discomfort, bloating, and alteration of bowel habits. It can be classified as either diarrhea-predominant, constipation-predominant or IBS with alternating stool pattern. As a functional bowel disorder, IBS has no known cause. Treatments can include different medications depending on the symptoms. Medications may consist of stool softeners and laxatives in constipation-predominant IBS, and antidiarrheals (e.g., opiate, opioid, or opioid analogs such as loperamide, codeine, diphenoxylate) in diarrhea-predominant IBS for mild symptoms and stronger opiates such as morphine and oxycodone for severe cases. Drugs affecting serotonin (5-HT) in the intestines can help reduce symptoms. Serotonin stimulates the gut motility and so agonists can help constipation-predominate irritable bowel, while antagonists can help diarrhea-predominant irritable bowel. Osmotic laxatives such as polyethylene glycol, sorbitol, and lactulose can help avoid "cathartic colon" which has been associated with stimulant laxatives. Lubiprostone (Amitiza), is a gastrointestinal agent used for the treatment of idiopathic chronic constipation and constipation-predominant IBS. Lubiprostone is a bicyclic fatty acid (prostaglandin E1 derivative) that acts by specifically activating C1C-2 chloride channels on the apical aspect of gastrointestinal epithelial cells, producing a chloride-rich fluid secretion. These secretions soften the stool, increase motility, and promote spontaneous bowel movements (SBM). The use of antispasmodic drugs (e.g., anticholinergics such as hyoscyamine or dicyclomine) may help patients, especially those with cramps or diarrhea. Any such therapeutic agents and methods can be used in conjunction with compositions comprising nadolol.

In some examples, provided herein is a composition comprising a therapeutically effective amount of nadolol for use in a method of treating collagenous or lymphocytic colitis in a human in need thereof wherein the nadolol is formulated for release in the small intestine or the large intestine of the human. Collagenous colitis is an inflammatory condition of the colon that cause persistent watery diarrhea. In lymphocytic colitis, the colonoscopy is normal but the mucosal biopsy reveals an accumulation of lymphocytes in the colonic epithelium and connective tissue (lamina propria). Both collagenous colitis and lymphocytic colitis are sometimes referred to collectively as microscopic colitis. The colonoscopy is normal but the mucosal biopsy reveals an accumulation of lymphocytes in the colonic epithelium and connective tissue and shows a distinctive thickening of the subepithelial collagen table. Treatment often begins with lifestyle changes. Budesonide (Entocort) is effective in controlling diarrhea in more than 75% of the patients with collagenous colitis. Any such therapeutic agents and methods can be used in conjunction with compositions comprising a nadolol.

In some examples, provided herein is a composition comprising a therapeutically effective amount of nadolol for use in a method of treating pancreatitis in a human in need thereof wherein the nadolol is formulated for release in the small intestine or the large intestine of the human. Pancreatitis is characterized by inflammation of the pancreas. It occurs when pancreatic enzymes that digest food are activated in the pancreas instead of the small intestine. It may be acute - beginning suddenly and lasting a few days, or chronic - occurring over many years. It has multiple causes and symptoms. The treatment of pancreatitis is supportive. Pain control is usually required. Morphine generally is suitable for pain control, among other medications. Oral intake, especially fats, is generally restricted at first. Fluids and electrolytes are replaced intravenously. However there is also evidence showing that earlier nutrition and feeding contributes to better recovery. The underlying cause should also be treated (targeting gallstones, discontinuing medications, cessation of alcohol etc.) The patient is monitored for complications. Endoscopic therapy by introducing stents to drain blocked pancreatic ducts Shock wave lithotripsy to pulverize pancreatic stones Surgery (laparoscopic and traditional), Islet cell transplantation may be offered if most or all of the pancreas is removed, Enzyme therapy for malabsorption helps restore normal digestion and reduces the amount of fat in the feces, leading to weight gain and improved well-being. Dietary changes such as eating smaller meals and limiting fats help reduce the need for digestive enzymes. Any such therapeutic agents and methods can be used in conjunction with compositions nadolol.

In some examples, provided herein is a composition comprising a therapeutically effective amount of nadolol for use in a method of treating reflux oesophagitis in a human in need thereof wherein the nadolol is formulated for release in the small intestine or the large intestine of the human. Reflux oesophagitis is inflammation of the esophagus which may be acute or chronic. The acute esophagitis can be catarrhal or phlegmonous, whereas the chronic esophagitis may be hypertrophic or atrophic. Causes of esophagitis include stomach acids backing up into the esophagus, infection, oral medications and allergies. Therapeutic agents include, but are not limited to, proton pump inhibitors which block acid production in the stomach and allow time for damaged esophageal tissue to heal. Drugs available by prescription include omeprazole (Prilosec), esomeprazole (Nexium) and lansoprazole (Prevacid). Over-the-counter proton pump inhibitors also are available. Other treatments for gastroesophageal reflux disease (GERD) may alleviate GERD symptoms temporarily, but generally have little effect on esophagitis. Fundoplication, a surgical procedure, may be used to treat GERD and improve the condition of the esophagus if other interventions don't work. During this procedure, a portion of the stomach is wrapped around the valve separating the esophagus and stomach (lower esophageal sphincter). This strengthens the sphincter and prevents acid from backing up into the esophagus. Fundoplication may also correct problems related to a hiatal hernia. Treatment for reflux esophagitis may also include avoiding alcohol, caffeine, aspirin, nonsteroidal anti-inflammatory medications, and foods that irritate your stomach. Any such therapeutic agents and methods can be used in conjunction with compositions comprising nadolol.

In some examples, the diseases to be treated using a composition comprising a therapeutically effective amount of nadolol are Irritable bowel syndrome, Irritable bowel syndrome variant of childhood with constipation, and Irritable bowel syndrome variant of childhood with diarrhea, According to the invention, the composition of nadolol is for use in treating IBS, wherein the nadolol is formulated for release in a large or a small intestine of a human.

In some examples, the diseases to be treated using a therapeutically effective amount of a composition comprising nadolol include gastrointestinal disease, inflammatory disease, inflammatory response, infectious disease, digestive system development and function, and hypersensitivity response.

The treatment of a condition described herein is effected by administration of a compound of the invention in conjunction with another therapeutic agent, adjuvant or therapeutic regimen. Regardless of the disease, disorder or condition being treated, the overall benefit experienced by the patient is additive of the two therapeutic agents or the patient experiences a synergistic benefit. Any agents that selectively regulate the expression or activity of the signaling pathways listed above can be used in the methods and compositions disclosed herein.

Provided herein are combination treatments using compositions comprising nadolol along with agents for the treatment of acidity related diseases such as antacids, which include magnesium compounds, aluminum compounds, calcium compounds, sodium compounds and combinations thereof. Acid reduction medications which are magnesium compounds include magnesium carbonate, magnesium oxide, magnesium peroxide, magnesium hydroxide, and magnesium silicate. Aluminum compounds include aluminum hydroxide, algeldrate, aluminum phosphate, dihydroxyaluminum sodium carbonate, aluminum acetoacetate, aloglutamol and aluminum glycinate. Calcium compounds include calcium carbonate and calcium silicate. Sodium compounds include sodium bicarbonate. Combinations and complexes of aluminum, calcium and magnesium compounds include ordinary salt combinations, magaldrate, almagate, hydrotalcite, and almasilate.

Other embodiments provided for are combination treatments using compositions comprising nadolol along with agents for treatment of peptic ulcer and gastro-oesophageal reflux disease which include: H2-receptor antagonists, prostaglandins, proton pump inhibitors, and combinations of agents for eradication of *Helicobacter pylori*, and other agents. H2-receptor antagonists include: cimetidine, ranitidine, famotidine, nizatidine, niperotidine, roxatidine, and lafutidine. Prostaglandins include agents such as misoprostol and enprostil. Proton pump inhibitors include omeprazole, pantoprazole, lansoprazole, rabeprazole, esomeprazole and dexlansoprazole. Combinations of agents for eradication of *Helicobacter pylori* include amoxicillin, clarithromycin and a proton pump inhibitor such as omeprazole, lansoprazole, pantoprazole or esomeprazole. Other agents for treatments of peptic ulcer and gastro-oesophageal reflux disease include: carbenoxolone, sucralfate, pirenzepine, methiosulfonium chloride, bismuth subcitrate, proglumide, sulglicotide, acetoxolone, zolimidine, troxipide, bismuth subnitrate, alginic acid, carbenoxolone, and combinations thereof, including combinations with psycholeptics and/or gefarnate.

Other embodiments provided for are combination therapies using compositions comprising nadolol with agents for the treatment of functional bowel diseases such as anticholinergics (e.g. with tertiary or quaternatry amino groups), antispasmodics, papaverine, drugs acting on serotonin receptors, Belladonna alkaloids, propulsive agents and derivatives and/or combinations thereof.

Anticholinergics include oxyphencyclimine, camylofin, mebeverine, trimebutine, rociverine, dicycloverine, dihexyverine, difemerine and piperidolate. Anticholinergics also include benzilone, glycopyrronium, oxyphenonium, penthienate, propantheline, otilonium bromide, methantheline, tridihexethyl, isopropamide, hexocyclium, poldine, mepenzolate, bevonium, pipenzolate, diphemanil, 2-benzhydryloxyethyl)diethyl-methylammonium iodide, tiemonium iodide, prifinium bromide, timepidium bromide, fenpiverinium, oxyphenonium, combinations and benzetimide carbachol and neostigmin. Synthetic antispasmodics include dimethylaminopropionylphenothiazine, nicofetamide and tiropramide. Papaverine and derivatives include papaverine, drotaverine and moxaverine. Drugs acting on serotonin receptors include alosetron, tegaserod, cilansetron, and prucalopride. Other agents used in the treatment of functional bowel diseases include fenpiprane, diisopromine, chlorbenzoxamine, pinaverium, fenoverine, idanpramine, proxazole, alverine, trepibutone, isometheptene, caroverine, phloroglucinol, silicones, trimethyldiphenylpropylamine, alverine, silicones, combinations, physiostigmin and macrogol ricinoleat. Belladonna alkaloids and derivatives thereof include atropine, hyoscyamine, belladonna total alkaloids, butylscopolamine, methylatropine, methylscopolamine, fentonium, and cimetropium bromide. Propulsive (prokinetic) agents include metoclopramide, cisapride, domperidone, bromopride, alizapride, clebopride and physiostigmine.

Other embodiments provided for are combination therapies using compositions comprising nadolol with agents such as laxatives, including agents such as softeners, emollients, contact laxatives, bulk producers, osmotically acting laxatives, enemas, peripheral opioid receptor antagonists and other laxatives. Softeners and emollients include liquid paraffin, docusate sodium and combinations thereof. Contact laxatives include oxyphenisatine, bisacodyl, dantron, phenolphthalein, castor oil, Senna glycosides, cascara, sodium picosulfate, bisoxatin, and combinations thereof. Bulk producers include ispaghula (psylla seeds), ethulose, sterculia, linseed, methylcellulose, triticum (wheat fibre), and polycarbophil calcium. Osmotically acting laxatives include magnesium carbonate, magnesium oxide, magnesium peroxide, magnesium sulfate, mineral salts, lactulose, lactitol, sodium sulfate, pentaerithrityl, macrogol, mannitol, sodium phosphate, sorbitol, magnesium citrate, sodium tartrate, lactulose and combinations thereof. Enemas include sodium phosphate, bisacodyl, dantron, glycerol, oil, sorbitol, docusate sodium, laurilsulfate and combinations thereof. Peripheral opioid receptor antagonists include methylnaltrexone bromide and alvimopan. Other laxatives include glycerol, carbon dioxide producing drugs and lubiprostone.

Other embodiments provided for are combination therapies using compositions comprising nadolol with agents such as intestinal anti-infective agents. Intestinal anti-infectives includeantibiotics and sulfonamides. Intestinal adsorbents include agents such as charcoal preparations, bismuth preparations, and other intestinal adsorbents. Intestinal anti-infectives also include antibiotics such as neomycin, nystatin, natamycin, streptomycin, polymyxin b, paromomycin, amphotericin b, kanamycin, vancomycin, colistin, rifaximin, neomycin, combinations, streptomycin, combinations, dihydrostreptomycin, gentamicin, apramycin, and bacitracin. Sulfonamides include phthalylsulfathiazole, sulfaguanidine, succinylsulfathiazole, sulfonamides, formosulfathiazole, phthalylsulfathiazole and combinations thereof.

Other embodiments provided for are combination therapies using compositions comprising nadolol with visilizumab, blinatumaomab, steroids, repertaxin, SB-265610, pioglitazone/metformin, rosiglitazone, GI262570, pioglitazone, tesaglitazar, troglitazone, interferon alfacon-1, interferon alfa-2a, interferon beta-la, IFNA2, interferon alfa-2b/ribavirin, pegintron, interferon beta-lb, flavopiridol, fingolimod, efalizumab, ampligen, amatuximab, alfecept, siplizumab, abatacept, or belatacept.

In some examples, surgical treatment is administered to a subject prior to, concurrent with or after administration of a composition comprising nadolol. Surgical treatments include proctosigmoidectomy, right colectomy, ileocolectomy, total abdominal colectomy, abdominoperineal resection, rectopexy, total proctocolectomy, endoscopic mucosal resection, vagotomy, pyloroplasty, and antrectomy.

In some examples, nadolol is used in animal models of gastrointestinal disease to inhibit and reduced the level or occurrence of inflammation, swelling, irritation, open sores, bleeding, ulcers, abdominal pain, immune response, and improvement with defecation in the subject. In some examples, the compound is used in animal models to investigate and determine the effectiveness of delivery systems or combination treatments with the compound.

In some examples, provided herein is a composition comprising a therapeutically effective amount of nadolol for use in a method of reducing or preventing fatigue, weight loss, loss of appetite, rectal bleeding, and loss of body fluids and electrolytes.

In some embodiments, a composition comprising nadolol for use in treating a gastrointestinal disease in a subject in need thereof is delivered by rectal administration.

In some embodiments, the pharmaceutical composition is chosen from the group consisting of enemas, rectal gels, rectal foams, rectal aerosols, suppositories, jelly suppositories, or retention enemas. In one embodiment, the composition is a suppository. In some embodiments, the composition is a rectal gel.

In some embodiments, a composition comprising nadolol for use in treating a gastrointestinal disease in a subject in need thereof is delivered by rectal administration, wherein the dose of nadolol is low.

In some embodiments, the low dose can be the absolute amount of nadolol the pharmaceutical composition comprises. For example, the pharmaceutical composition can comprise less than 70, 60, 50, 40, 30, 20, 15, 10, 8, 6, 4, 3, 2, 1, 0.5, 0.4, 0.1 mg of beta blocker. In some embodiments, the pharmaceutical composition comprises less than 40, 20, 10, 8, 6, 4, or 2 mg of beta blocker. In some embodiments, the pharmaceutical composition comprises less than 40, 20, 10, 8, 6, 4, or 2 mg of beta blocker and greater than 0.05, 0.1, 0.2, 0.3, 0.4, 0.5, 0.8, 1.0, 1.3, 1.6, or 1.9 mg of beta blocker. In some embodiments, the pharmaceutical composition comprises between about 0.1 mg and about 20 mg of beta blocker.

In some embodiments, the low dose can be the amount of beta blocker the pharmaceutical composition comprises relative to the subject's body weight. In other embodiments, the low dose is determined by the absolute amount of beta blocker that the subject is administered. For example, the subject can be administered less than 40, 20, 10, 8, 6, 4, or 2 mg of beta blocker daily. The subject can be administered less than 40 mg of beta blocker daily. The subject can be administered less than 20 mg of beta blocker daily. The subject can be administered less than 10 mg of beta blocker daily.

In other embodiments, the low dose is determined by the amount of beta blocker that the subject is administered relative to the subject's body weight. For example, the subject can be administered less than 0.6, 0.4, 0.2, 0.1, 0.05, 0.02, or 0.01 mg of beta blocker daily per kg of the subject's body weight. In some embodiments, the subject is administered less than 0.6 mg of beta blocker per kg of the subject's body weight daily. In other embodiments, the subject is administered less than 0.2 mg of beta blocker per kg of the subject's body weight daily. In other embodiments, the subject is administered less than 0.1 mg of beta blocker per kg of the subject's body weight daily. In some embodiments, the subject is administered between about 0.05 mg and about 1 mg of beta blocker per kg of the subject's body weight daily.

In other embodiments, the amount of beta blocker can be correlated with the pharacokinetics of the beta blocker. For example, the amount of beta blocker in the pharmaceutical composition can be selected such that administration of the pharmaceutical composition results in a peak plasma level in the subject of less than 50, 40, 30, 20, or 10 ng/mL. In some embodiments, the amount of beta blocker in the pharmaceutical composition can be selected such that administration of the pharmaceutical composition results in a peak plasma level in the subject of greater than 1, 2, 4, 6, 8, 10, 14, 18, 20, or 25 ng/mL. In some embodiments, the amount of beta blocker in the pharmaceutical composition can be selected such that administration of the pharmaceutical composition results in a peak plasma level in the subject of between about 1 ng/mL and about 50 ng/mL.

The some embodiments, wherein the pharmaceutical composition comprises an amount of beta blocker, which, when administered to the subject orally, is insufficient to result in an improvement in the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject. In some embodiments, the pharmaceutical composition comprises an amount of beta blocker which is sufficient to result in an improvement in the intestinal barrier in the large or small intestine of said subject, and wherein said improvement comprises a reduction in the level of or number of occurrences of inflammation, swelling, irritation, open sores, bleeding, ulcers, abdominal pain and defecation irregularities in the subject.

The severity of IBD can be determined by any clinically validated method. For instance, the severity of IBD can be measured by analysis of the bowel. In some embodiments, the severity of IBD is assessed using the following scoring system: maximum score = 10

| 1) | Adhesions: |
|---|---|
| | none = 0 |
| | minimal = 1 |
| | involving several bowel loops = 2 |
| | |

| 2) | Strictures: |
|---|---|
| | none = 0 |
| | mild = 1 |
| | moderate = 2 |
| | severe, proximal dilatation = 3 |
| | |

| 3) | Ulcers: |
|---|---|
| | none = 0 |
| | inear ulceration < 1 cm = 1 |
| | two linear ulcers < 1 cm = 2 |
| | more sites of ulceration or one large ulcer = 3 |
| | |

| 4) | Wall thickness: |
|---|---|
| | less than 1 mm = 0 |
| | 1-3 mm = 1 |
| | > 3 mm = 2 |

The severity of IBD can be determined by histopatholgy of the bowel. For instance, examinign the cells of the bowel for inflammation or necrosis can be a method for determining the severity of IBD. In some embodiments, the severity of IBD can be determined by scoring inflammation and necrosis of the colon by a scoring method. One such scoring method is:
*Inflammation* - neutrophil and macrophage infiltration predominantly, fibroplasia and neovascularization in areas of transmural necrosis
   0= No inflammation
   0.5= Very minimal focal infiltrates in mucosa only, affects <2% of the total colon
   1.0= Minimal multifocal infiltrates in mucosa only, affects 2- 10% of the total colon
   2.0= Mild multifocal infiltrates affecting mucosa, submucosa, outer muscle layers and serosa, affects 11-25% of the total colon
   3.0= Moderate multifocal infiltrates affecting mucosa, submucosa, outer muscle layers and serosa, affects 26-50% of the total colon
   4.0= Marked multifocal to diffuse infiltrates affecting mucosa, submucosa, outer muscle layers and serosa, affects 51-75% of the total colon
   5.0= Severe multifocal to diffuse infiltrates affecting mucosa, submucosa, outer muscle layers and serosa, affects >75% of the total colon.
*Necrosis* - measure the total length of all colon sections, measure the total length of necrotic mucosa devoid of epithelium to determine % area affected
   0= No necrosis
   0.5= Very minimal, focal, affects <2% of the total colon
   1.0= Minimal focal or multifocal, affects 2-10% of the total colon
   2.0= Mild, focal or multifocal, affects 11-25% of the total colon
   3.0= Moderate focal or multifocal, affects 26-50% of the total colon
   4.0= Marked focal or multifocal, affects 51-75% of the total colon
   5.0= Severe, affects >75% of the total colon

While preferred embodiments of the present invention have been shown and described herein, it will be obvious to those skilled in the art that such embodiments are provided by way of example only.

### EXAMPLES

### 1: Pharmaceutical Compositions

### Example 1a: Parenteral Composition

To prepare a parenteral pharmaceutical composition suitable for administration by injection, 80 mg of a compound of Formula I, described herein is dissolved in DMSO and then mixed with 10 mL of 0.8% sterile saline. The mixture is incorporated into a dosage unit form suitable for administration by injection.

### Example 1b: Oral Composition

To prepare a pharmaceutical composition for oral delivery, 80 mg of a compound of Formula I, described herein is mixed with 8000 mg of starch. The mixture is incorporated into an oral dosage unit for, such as a hard gelatin capsule, which is suitable for oral administration.

### Example 1c: Sublingual (Hard Lozenge) Composition

To prepare a pharmaceutical composition for buccal delivery, such as a hard lozenge, mix 80 mg of a compound of Formula I, described herein, with 500 mg of powdered sugar mixed, with 1.6 mL of light corn syrup, 2.4 mL distilled water, and 0.42 mL cherry extract. The mixture is gently blended and poured into a mold to form a lozenge suitable for buccal administration.

### Example 1d: Inhalation Composition

To prepare a pharmaceutical composition for inhalation delivery, 25 mg of a compound of Formula I, described herein is mixed with 50 mg of anhydrous citric acid and 150 mL of 0.9% sodium chloride solution. The mixture is incorporated into an inhalation delivery unit, such as a nebulizer, which is suitable for inhalation administration.

### Example 1e: Rectal Gel Composition

To prepare a pharmaceutical composition for rectal delivery, 1500 mg of a compound of Formula I, described herein is mixed with 2.0 g of PVP 90, 100 mg of methylparapen, 5 g of glycerin and 100 mL of purified water. The resulting gel mixture is then incorporated into rectal delivery units, such as syringes, which are suitable for rectal administration.

### Example If: Topical Gel Composition

To prepare a pharmaceutical topical gel composition, 80 mg of a compound of a compound of Formula I, described herein is mixed with 2.0 g of PVP 90, 10 mL of propylene glycol, 10 mL of isopropyl myristate and 100 mL of purified alcohol USP. The resulting gel mixture is then incorporated into containers, such as squeeze bottles, which are suitable for topical administration.

### Example 1g: Ophthalmic Solution Composition

To prepare a pharmaceutical ophthalmic solution composition, 80 mg of a compound of Formula I, described herein is mixed with 0.9 g of NaCl in 100 mL of purified water and filtered using a 0.1 micron filter. The resulting isotonic solution is then incorporated into ophthalmic delivery units, such as eye drop containers, which are suitable for ophthalmic administration.

### Example 2

Prophylactic efficacy study in TNBS (2,4,6-trinitrobenzenesulfonic acid) rat colitis model.

Preparation of doses: For Groups 1, 2: 50 ml 0.1M acetic acid and 30 ml sterile water were combined. The pH was adjusted from 4.45 to 6.7 with 0.1N NaOH. The stir bar was removed and the solution made up to 100 ml with sterile water. For Group 3, 133.9 mg prednisolone 21-hemisuccinate sodium salt was dissolved in 100 ml de-ionized water to prepare a 1 mg/ml solution. For Group 4, 600 mg Nadolol was dissolved in 50 ml 0.1M acetic acid and 30 ml sterile water. The pH was adjusted from 4.45 to 6.7 with 0.1N NaOH, and the solution made up to 100 ml with sterile water. For Group 5, 1.2 g Nadolol was dissolved in 50 ml 0.1M acetic acid and 30 ml sterile water. The pH was adjusted from 4.45 to 6.7 with 0.1N NaOH and the solution made up to 100 ml with sterile water. For Group 6: 1 g cellulose gum was dissolved in 50 ml 0.1N acetic acid and 30 ml de-ionized water. The volume was made up to 100 ml with sterile water. For Group 7, 600 mg Nadolol was dissolved in 50 ml 0.1M acetic acid and 30 ml sterile water. 1 g cellulose gum was added and dissolved into the solution and the solution made up to 100 ml with sterile water. For Group 8, 1.2 g Nadolol was dissolved in 50 ml 0.1M acetic acid and 30 ml sterile water. 1 g cellulose gum was added and dissolved into the solution and the solution made up to 100 ml with sterile water.

**Table 2: Treatment Groups**

| Group | Treatment | Dose (mg/kg) | ROA |
|---|---|---|---|
| 1 | Vehicle | 10 ml/kg | PO |
| 2 | Vehicle | 10 | PO |
| 3 | Prednisolone | 10 | PO |
| 4 | Nadolol | 60 | PO |
| 5 | Nadolol | 120 | PO |
| 6 | Vehicle | 10 ml/kg | IR |
| 7 | Nadolol | 60 | IR |
| 8 | Nadolol | 120 | IR |

(Day -1) 84 Sprague-Dawley rats (male, 160-180 g each) were sorted into treatment groups based upon average body weight. The rats were weighed and food-deprived. The rats in groups 1-5 were dosed orally (PO) at 10 ml/kg with their respective solutions according to Table 2. The rats in Groups 6-8 were anesthetized intra-rectally (IR) instilled with 10 ml/kg of their respective treatment according to Table 2, the anus was pinched closed and the rat held inverted until recovered from anesthesia (at least 1 min).

After 24 hours (day 0), an aqueous solution of TNBS (2,4,6-trinitrobenzenesulfonic acid) 16 mg/ml in 50% ethanol was made and the rats were weighed and dosed as in TABLE 2. One hour later, the rats anesthetized and Groups 2-8 were intra-rectally instilled with 4 ml/kg TNBS solution (64 mg/kg), the anus was pinched closed and the rat held inverted until recovered from anesthesia (at least 1 min). Group 1 received de-ionized water. The rats were re-fed after recovery from TNBS instillation.

The rats were weighed and dosed as in TABLE 2 each day for the next six days (day 2-7), The rats were anesthetized and exsanguinated into pre-chilled serum separator tubes, the blood processed to serum which was stored in several aliquots at -80 °C. A midline incision was made in the abdomen and the colon was evaluated for adhesions and stricture. The colon was removed and the length recorded. A midline incision was made the entire length of the colon, the contents removed and the colon weight and colon wall thickness recorded. A section of the colon was preserved in 20 volumes of 10% neutral buffered formalin. The end of the colon section at the rectal end of the section was edged with Evans Blue dye for orientation purposes. Another section of the colon was weighed and homogenized on an ice batch in 5 ml PBS. The homogenate was centrifuged and aliquots stored in labeled Eppendorf tubes at -80 °C. The following parameters were used to determine colonic score:

**Colonic Score Parameters**

| a) | Adhesions: | |
|---|---|---|
| | 1) | none = 0 |
| | 2) | minimal = 1 |
| | 3) | involving several bowel loops = 2 |

| b) | Strictures: | |
|---|---|---|
| | 1) | none = 0 |
| | 2) | mild = 1 |
| | 3) | moderate = 2 |
| | 4) | severe, proximal dilatation = 3 |

| c) | Ulcers: | |
|---|---|---|
| | 1) | none = 0 |
| | 2) | linear ulceration < 1 cm = 1 |
| | 3) | two linear ulcers < 1 cm = 2 |
| | 4) | more sites of ulceration or one large ulcer = 3 |

| d) | Wall thickness: | |
|---|---|---|
| | 1) | less than 1 mm = 0 |
| | 2) | 1-3 mm = 1 |
| | 3) | > 3 mm = 2 |

The formalin-preserved colon samples were submitted for histological preparation and evaluation. Sections of H&E stained colon section were analyzed using criteria below and scored for inflammation and necrosis.

*Inflammation* - neutrophil and macrophage infiltration predominantly, fibroplasia and neovascularization in areas of transmural necrosis.
0= No inflammation
0.5= Very minimal focal infiltrates in mucosa only, affects <2% of the total colon
1.0= Minimal multifocal infiltrates in mucosa only, affects 2- 10% of the total colon
2.0= Mild multifocal infiltrates affecting mucosa, submucosa, outer muscle layers and serosa, affects 11-25% of the total colon
3.0= Moderate multifocal infiltrates affecting mucosa, submucosa, outer muscle layers and serosa, affects 26-50% of the total colon
4.0= Marked multifocal to diffuse infiltrates affecting mucosa, submucosa, outer muscle layers and serosa, affects 51-75% of the total colon
5.0= Severe multifocal to diffuse infiltrates affecting mucosa, submucosa, outer muscle layers and serosa, affects >75% of the total colon.

*Necrosis* - measure the total length of all colon sections, measure the total length of necrotic mucosa devoid of epithelium to determine % area affected
0= No necrosis
0.5= Very minimal, focal, affects <2% of the total colon
1.0= Minimal focal or multifocal, affects 2-10% of the total colon
2.0= Mild, focal or multifocal, affects 11-25% of the total colon
3.0= Moderate focal or multifocal, affects 26-50% of the total colon
4.0= Marked focal or multifocal, affects 51-75% of the total colon
5.0= Severe affects >75% of the total colon

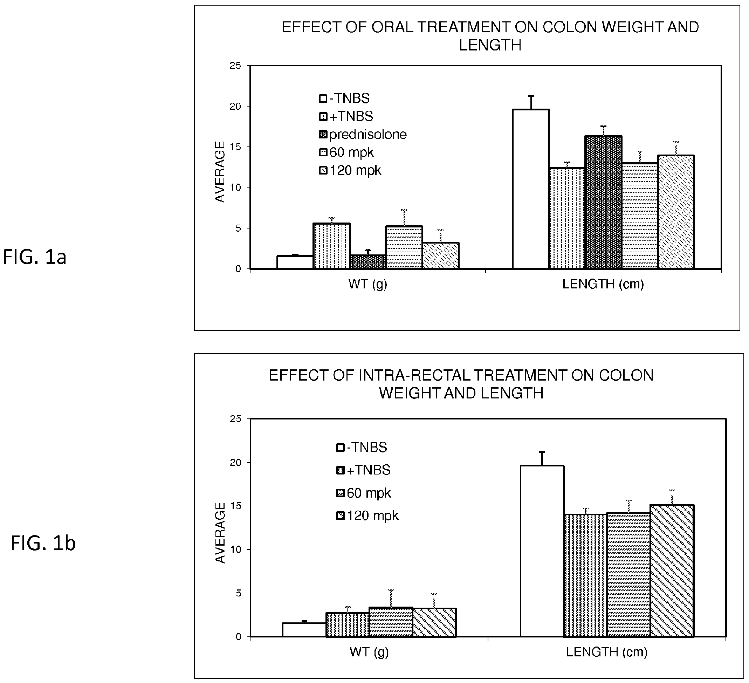

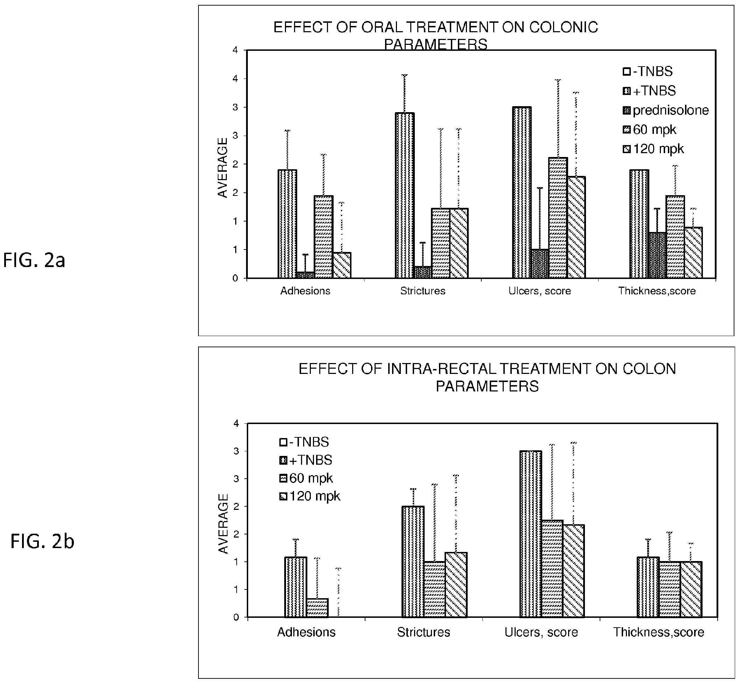

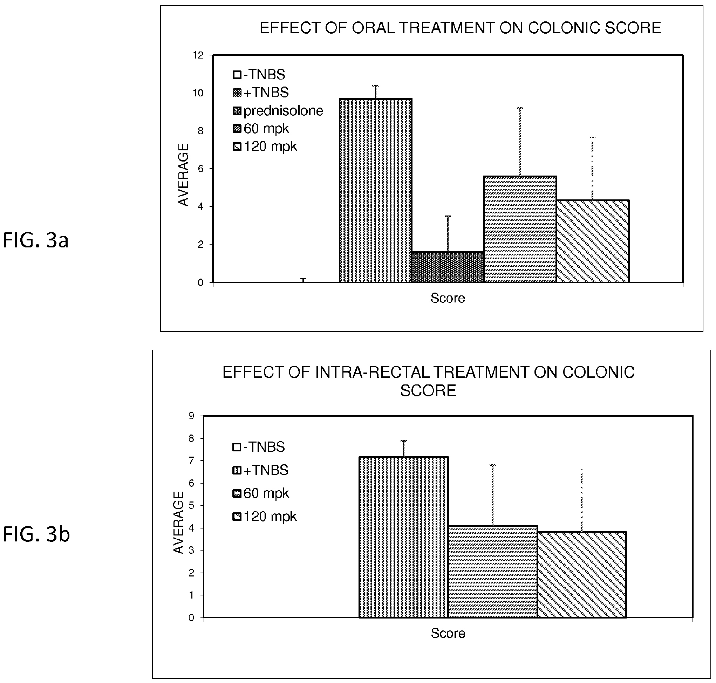

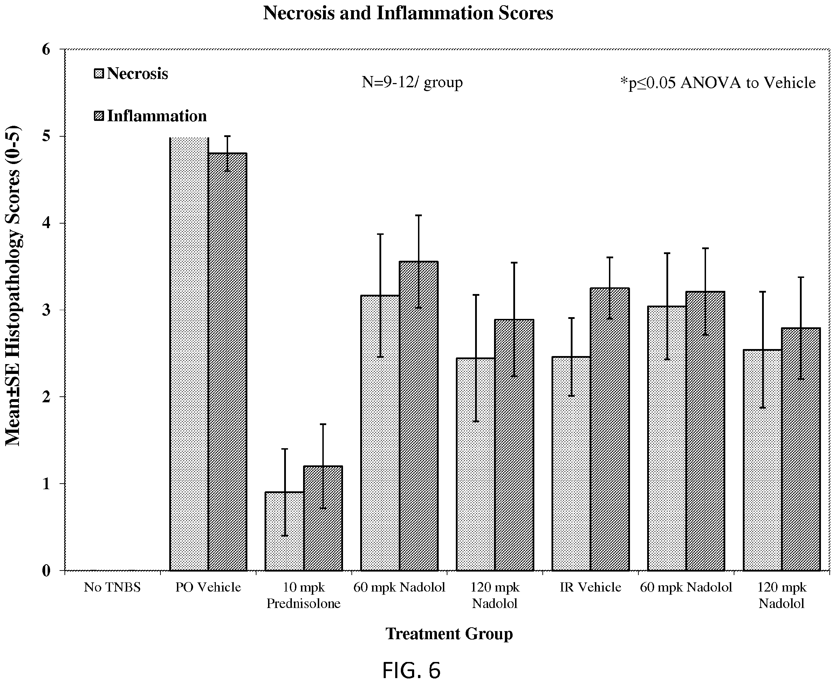

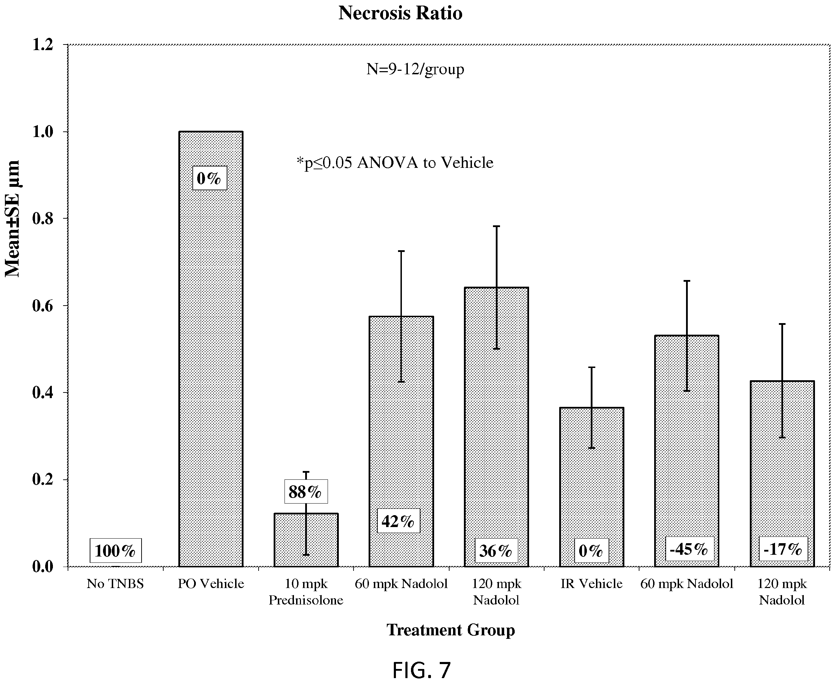

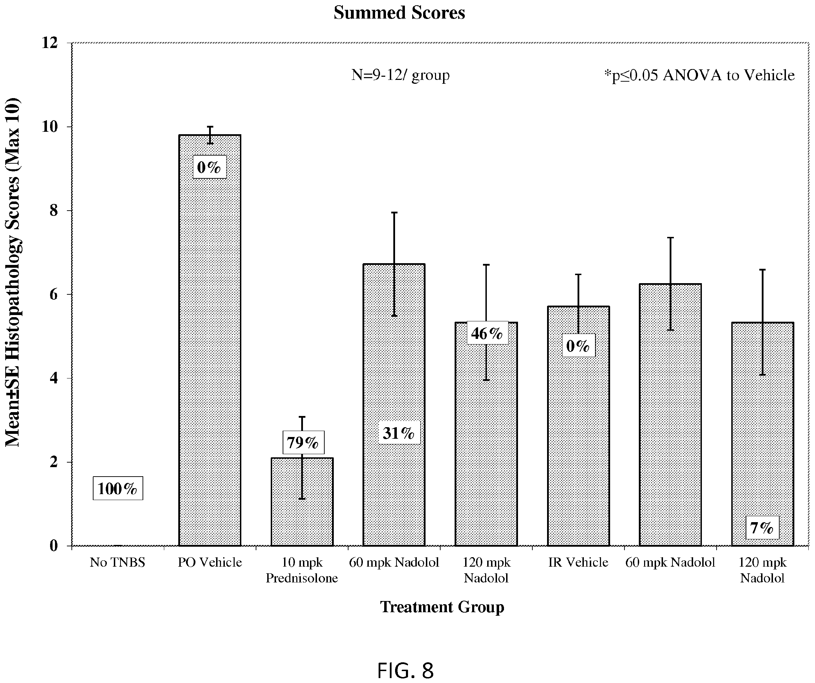

The results of the study are summarized graphically in Figures 1-8.

### Example 3

TNBS Dose Escalation Study

Sprague-Dawley (SD) rats 118 (male, 180-200 g) were assigned to 11 groups of 10 or 12 rats per group so that each group has approximately the same mean weight.

(Day -1) Initiate daily dosing of Groups 1-11 as in TABLE 1. Rats are deprived of food for 24 hours prior to disease initiation. 1) Intra-rectal (IR) administration is accomplished by briefly anesthetizing the rats, administering the test material at 10 ml/kg via a 20 gauge feeding needle inserted via the rectum, maintaining the rat in an inverted position with anus pinched shut until recovered from anesthesia. 2) Oral formulations are first dissolved in 0.05N acetic acid and pH adjusted to 6-7 with 0.1N NaOH.

**Table 3: Treatment groups**

| Group | # rats | Treatment | Dose (mg/kg) | ROA |
|---|---|---|---|---|
| 1 | 10 | Vehicle | 10 ml/kg | PO |
| 2 | 10 | Vehicle | 10 ml/kg | PO |
| 3 | 10 | Prednisolone | 1 | PO |
| 4 | 10 | Nadolol | 6 | PO |
| 5 | 10 | Nadolol | 19 | PO |
| 6 | 10 | Nadolol | 60 | PO |
| 7 | 10 | Nadolol | 120 | PO |
| 8 | 12 | Vehicle | 10 ml/kg | IR |
| 9 | 12 | Nadolol | 6 | IR |
| 10 | 12 | Nadolol | 19 | IR |
| 11 | 12 | Nadolol | 60 | IR |

| | | | | |
|---|---|---|---|---|
| *PO = oral gavage **IR = intra-rectal | | | | |

(DAY 0) Prepare TNBS Solution (50% TNBS: 50% 200 proof ethanol; 16 mg/ml TNBS).
Dose as in TABLE 3. After 1 hour, rats in Groups 2-11 are administered TNBS. Anesthetize rats and administer 64 mg/kg (4 ml/kg) TNBS Solution into colon of each rat, then re-feed rats.

(DAY 7): Rats are weighed and dosed as in Table 3. Rats are weighed and dosed as in TABLE 3. Terminal blood samples are collected from anesthetized rats. Samples are collected in pre-chilled-tubes and processed to serum. Store serum at -80 °C in several aliquots. A midline incision is made on each animal. Colon is removed and length measured and recorded. A longitudinal incision is made on each colon. Fecal material is gently washed away with saline. Excess fluid is gently blotted and colon weighed. The severity of IBD is assessed using the following scoring system: maximum score = 10

| 1) | Adhesions: |
|---|---|
| | none = 0 |
| | minimal = 1 |
| | involving several bowel loops = 2 |
| | |

| 2) | Strictures: |
|---|---|
| | none = 0 |
| | mild = 1 |
| | moderate = 2 |
| | severe, proximal dilatation = 3 |
| | |

| 3) | Ulcers: |
|---|---|
| | none = 0 |
| | inear ulceration < 1 cm = 1 |
| | two linear ulcers < 1 cm = 2 |
| | more sites of ulceration or one large ulcer = 3 |

| 4) | Wall thickness: |
|---|---|
| | less than 1 mm = 0 |
| | 1-3 mm = 1 |
| | > 3 mm = 2 |

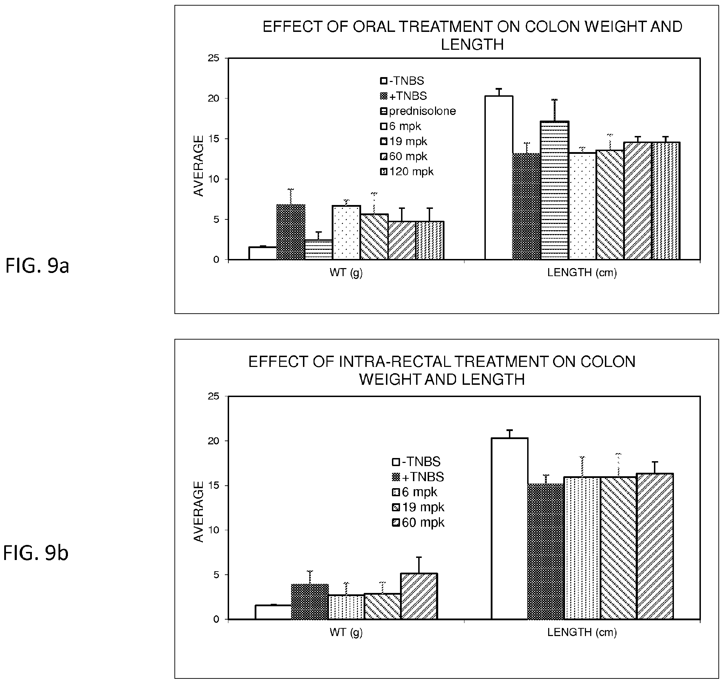

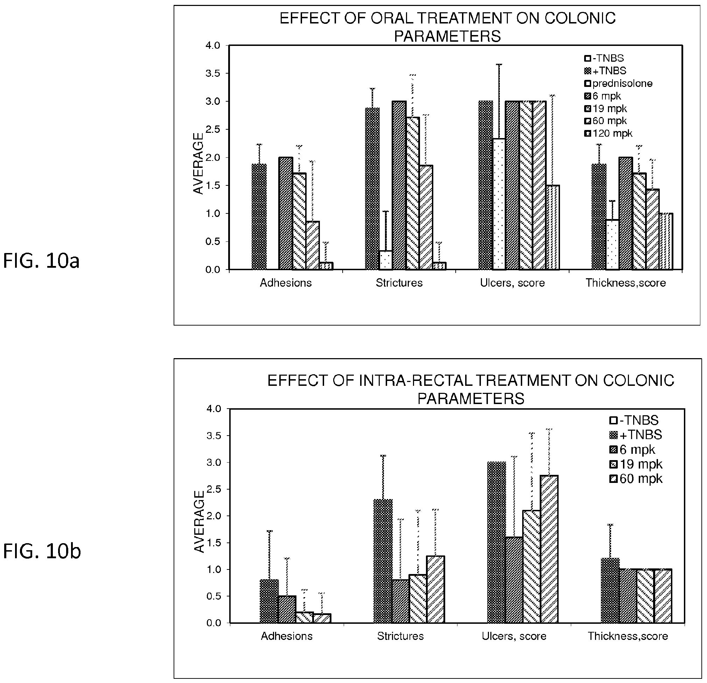

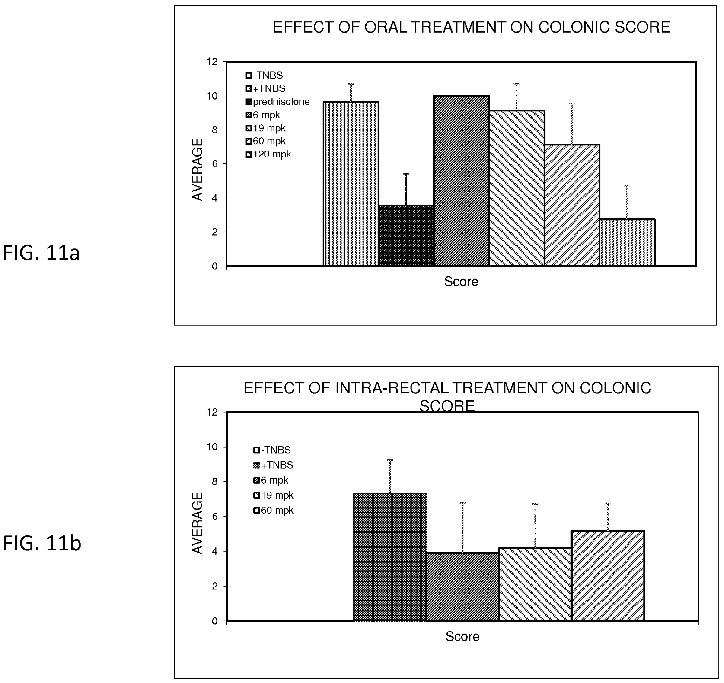

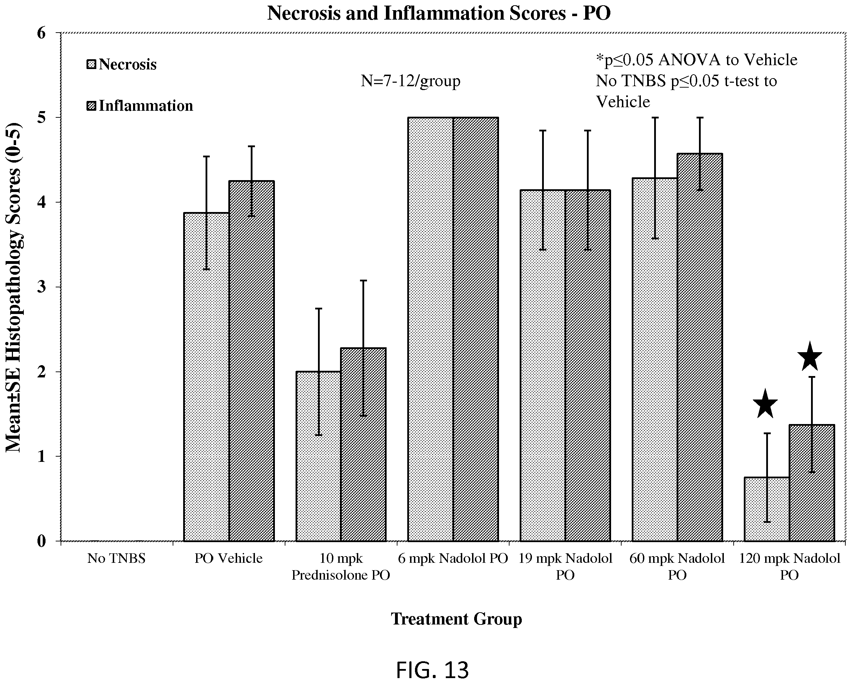

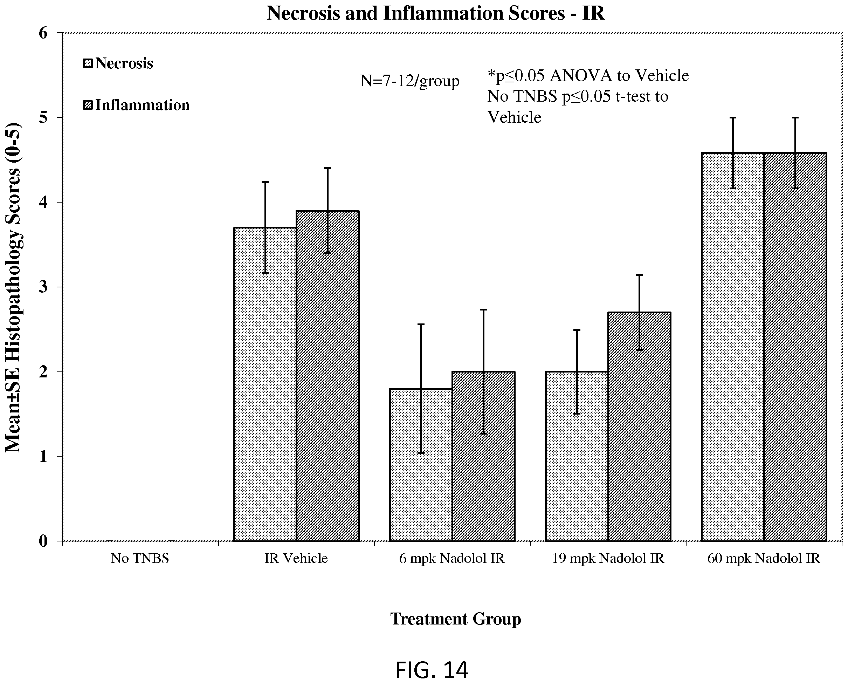

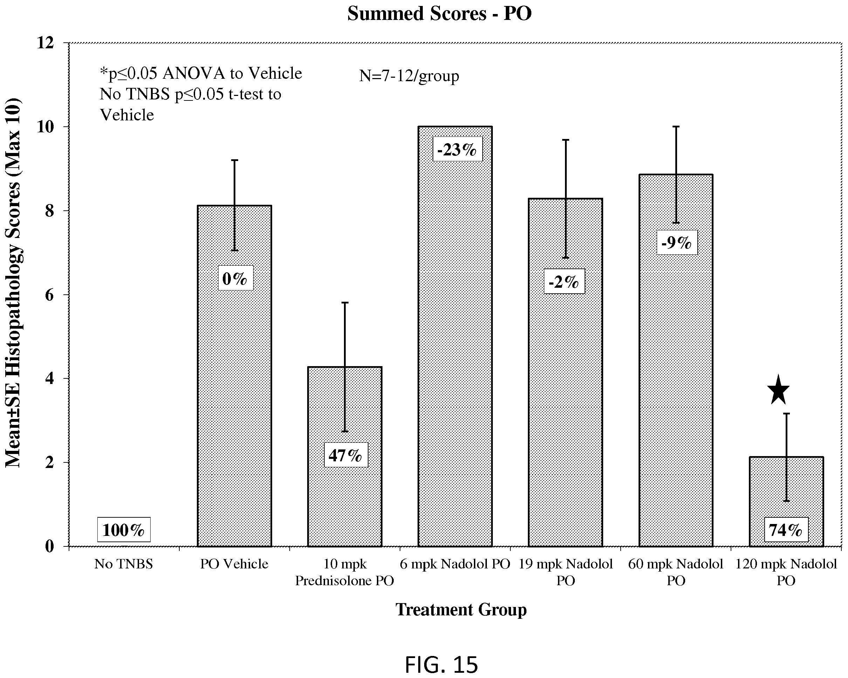

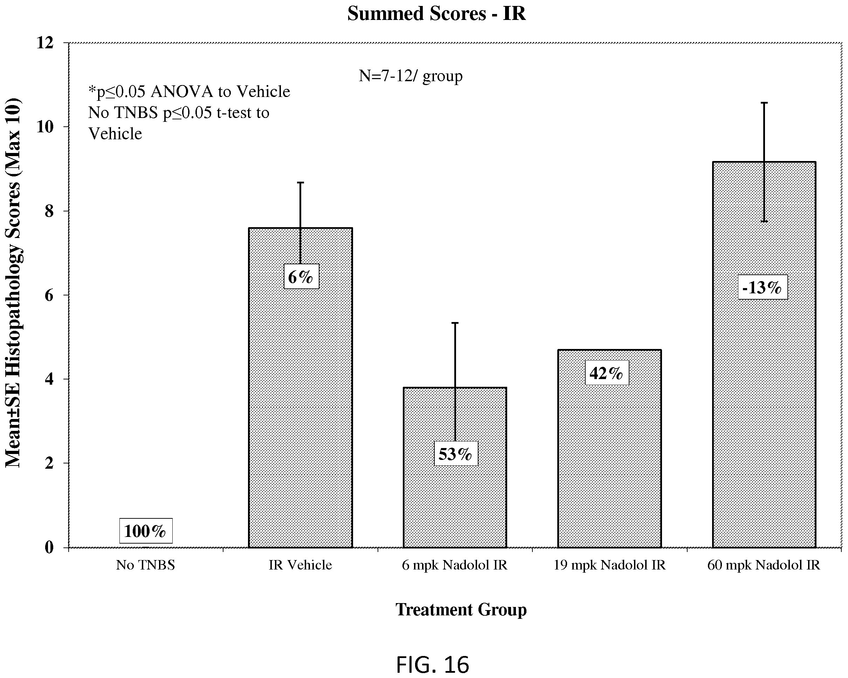

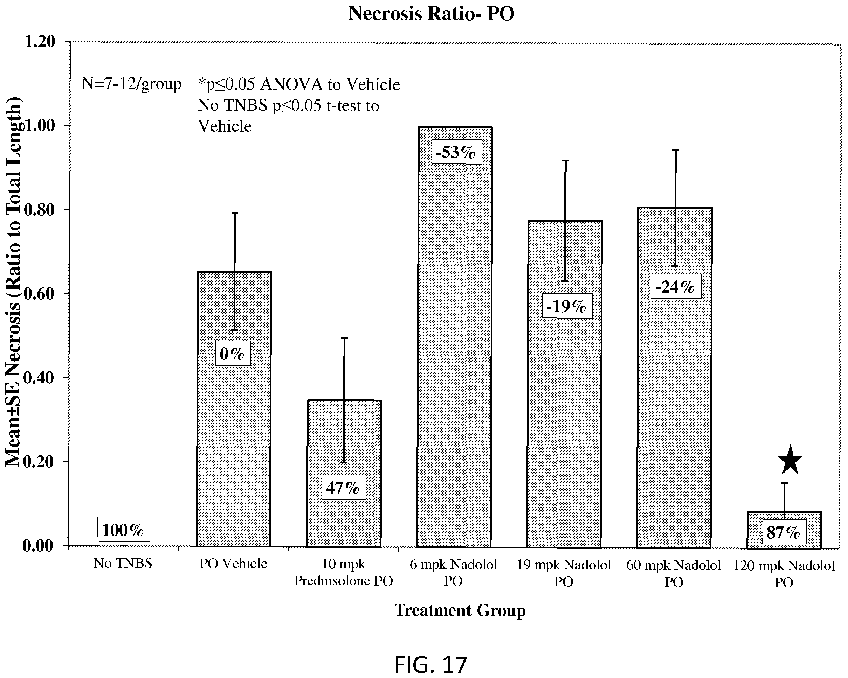

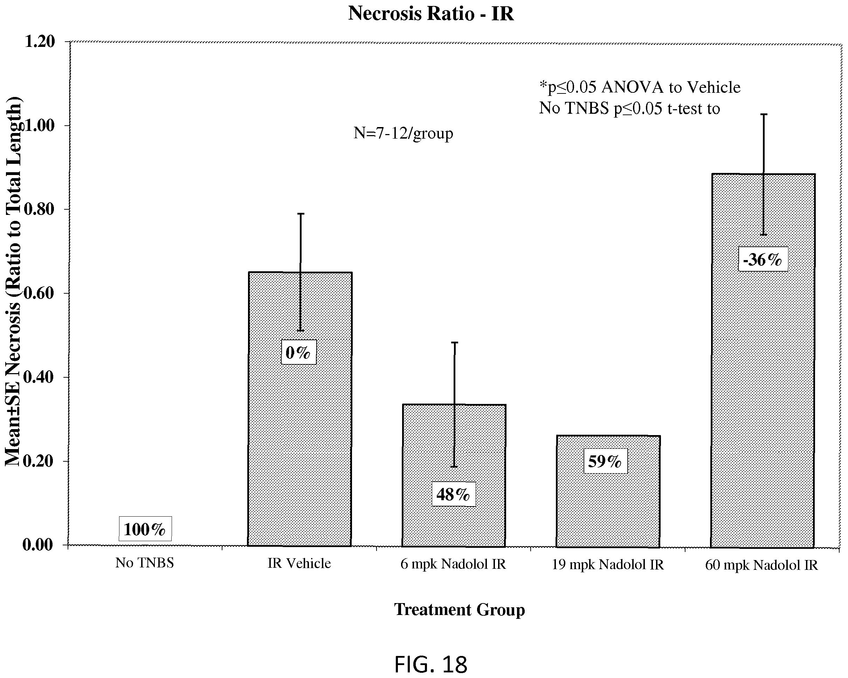

A section of colon is preserved in 10 volumes of 10% neutral buffered formalin for histopathology. A section of colon is homogenized in PBS, centrifuged, and aliquots of the homogenate in labeled Eppendorf tubes are stored at -80 °C for cytokine analysis.
The results of this study were recorded in displayed in figures 9-18.

### Example 4

Pharmakokinetics of Nadolol was determined in diseased and healthy rats.

48 Sprague-Dawley rats (male, 180-200 g) were assigned to 8 groups of 6 rats per group so that each group has approximately the same mean weight.

(DAY -1) Rats were deprived of food 24 hours prior to disease initiation.

(DAY 0) TNBS Solution (50% TNBS/50% 200 proof ethanol; 16 mg/ml TNBS) was prepared. Rats in Groups 3, 4, 7 and 8 were administered TNBS: rats were anesthetized and administered 64 mg/kg (4 ml/kg) TNBS Solution into colon of each rat then rats were refed.

(DAY 6) Rats were bled for pre-dose samples by retro-orbital bleeds. Rats were weighed and dosed as in TABLE 4. Intra-rectal (IR) administration was accomplished by briefly anesthetizing rats, administering the test material at 10 ml/kg via a feeding needle inserted via the rectum, maintaining the rat in an inverted position with anus pinched shut until recovered from anesthesia. Oral formulations (PO) were first dissolved in 0.05N acetic acid and pH adjusted to 6-7 with 0.1N NaOH.

**Table 4: Treatment Regimen**

| Group | # rats | Treatment | Dose (mg/kg) | ROA |
|---|---|---|---|---|
| 1 | 6 | NOTNBS Nadolol | 19 | PO |
| 2 | 6 | NO TNBS Nadolol | 120 | PO |
| 3 | 6 | TNBS Nadolol | 19 | PO |
| 4 | 6 | TNBS Nadolol | 120 | PO |
| 5 | 6 | NO TNBS Nadolol | 19 | IR |
| 6 | 6 | NO TNBS Nadolol | 120 | IR |
| 7 | 6 | TNBS Nadolol | 19 | IR |
| 8 | 6 | TNBS Nadolol | 120 | IR |

| | | | | |
|---|---|---|---|---|
| *PO = oral gavage **IR = intra-rectal | | | | |

Blood samples were collected from rats by retro-orbital bleed at time points: 30, 60, 90, 120, 180, 240 and 360 minutes post-dose. Samples were collected in pre-chilled-tubes and processed to EDTA plasma. The plasma was aliquoted to four aliquots and stored immediately at -80°C. Each aliquot (50-100 uL) was warmed to room temperature and the plasma level of Nadolol was measured. The results of this pharmacokinetic study are presented in Figure 19.

### Example 5

### Oral Beta Blocker Efficacy Study.

80 Sprague-Dawley (SD) rats (male, 180-200g) were assigned to 7 groups of 10-12 rats per group so that each group had approximately the same mean weight.

(Day -1): Groups 1-7 were dosed as described in TABLE 5.

**Table 5: Group Treatments**

| Group | # rats | Treatment | Dose (mg/kg) | ROA |
|---|---|---|---|---|
| 1 | 10 | Vehicle/NO TNBS | 10 ml/kg | PO |
| 2 | 12 | Vehicle | 10 ml/kg | PO |
| 3 | 12 | Prednisolone | 10 | PO |
| 4 | 12 | Nadolol | 120 | PO |
| 5 | 12 | Timolol maleate | 120 | PO |
| 6 | 12 | Propranolol HCl | 120 | PO |
| 7 | 12 | Betaxolol | 120 | PO |

| | | | | |
|---|---|---|---|---|
| PO = oral gavage | | | | |

Rats were deprived of food for 24 hours prior to disease initation on Day 0.
Day 0: TNBS solution (50% TNBS: 50% 200 proof ethanol; 16 mg/ml TNBS). Rats in groups 2-8 were administered TNBS solution (Group 1 is NOT treated with TNBS): rats anesthetized, feeding needle inserted into rat anus, 64 mg/kg (4 mL/kg) TNBS solution added to colon of each rat, feeding needle removed, anus closed, rat maintained head down position until recovery from anesthesia, then rats re-fed.
Animals were dosed once daily DAYS -1-7 according to the group treatments listed in Table 5.

On DAY 7, Rats were dosed as in TABLE 5, then blood samples were collected from the anesthetized rats. A midline incision was made on each animal, the colon was removed and length measured and recorded, a longitudinal incision was made on each colon, fecal material was gently washed away with saline, excess fluid was gently blotted and the colon weighed. The severity of IBD was assessed using the following scoring system (maximum score = 10):

| 1) | Adhesions: |
|---|---|
| | none = 0 |
| | minimal = 1 |
| | involving several bowel loops = 2 |

| 2) | Strictures: |
|---|---|
| | none = 0 |
| | mild = 1 |
| | moderate = 2 |
| | severe, proximal dilatation = 3 |

| 3) | Ulcers: |
|---|---|
| | none = 0 |
| | inear ulceration < 1 cm = 1 |
| | two linear ulcers < 1 cm = 2 |
| | more sites of ulceration or one large ulcer = 3 |

| 4) | Wall thickness: |
|---|---|
| | less than 1 mm = 0 |
| | 1-3 mm = 1 |
| | > 3 mm = 2 |

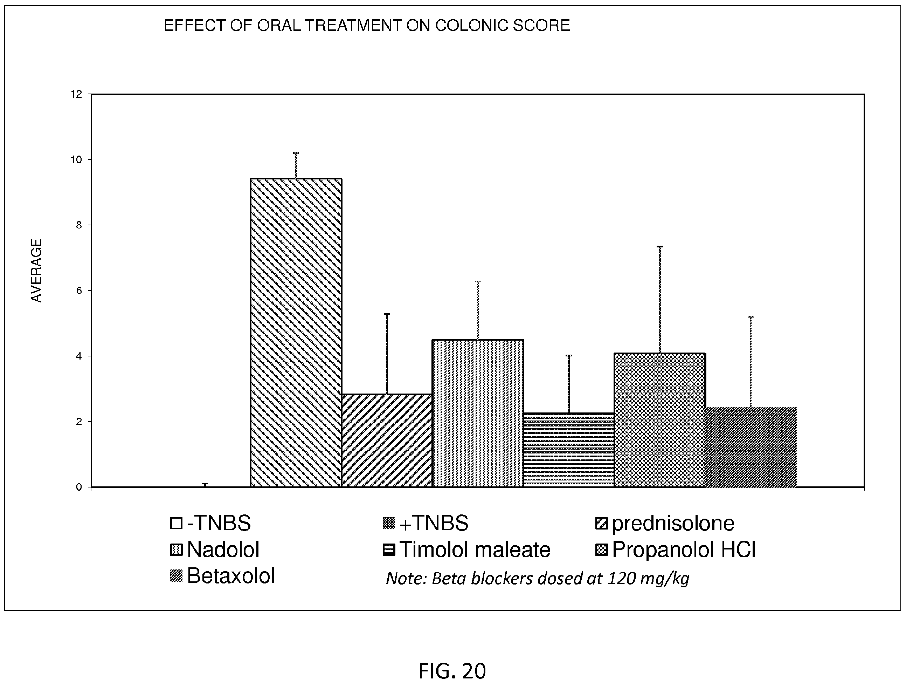

A section of colon was preserved in 10 volumes of 10% neutral buffered formalin for histopathology. A section of the colon is homogenized in PBS, centrifuged, and aliquots of the homogentat were frozen and stored for cytokine analysis. The results of this study were recorded and displayed in FIG. 20.

### Example 6

A Rectal Beta Blocker Efficacy Study was conducted.

80 Sprague-Dawley (SD) rats (male, 180-200g) were assigned to 7 groups of 10-12 rats per group so that each group had approximately the same mean weight.

(Day -1): Groups 1-7 were dosed as described in TABLE 6.

**Table 6: Group Treatments**

| Group | # rats | Treatment | Dose (mg/kg) | ROA |
|---|---|---|---|---|
| 1 | 10 | Vehicle/NO TNBS | 10 ml/kg | IR |
| 2 | 12 | Vehicle | 10 ml/kg | IR |
| 3 | 12 | Prednisolone | 10 | PO |
| 4 | 12 | Nadolol | 6 | IR |
| 5 | 12 | Timolol maleate | 6 | IR |
| 6 | 12 | Propranolol HCl | 6 | IR |
| 7 | 12 | Betaxolol | 6 | IR |

| | | | | |
|---|---|---|---|---|
| PO = oral gavage IR = intra-rectal administration under anesthesia | | | | |

Rats were deprived of food for 24 hours prior to disease initation on Day 0.

Day 0: TNBS solution (50% TNBS: 50% 200 proof ethanol; 16 mg/ml TNBS). Rats in groups 2-8 were administered TNBS solution (Group 1 is NOT treated with TNBS): rats anesthetized, feeding needle inserted into rat anus, 64 mg/kg (4 mL/kg) TNBS solution added to colon of each rat, feeding needle removed, anus closed, rat maintained head down position until recovery from anesthesia, then rats re-fed.

Animals were dosed once daily DAYS -1-7 according to the group treatments listed in Table 6.

On DAY 7, Rats were dosed as in TABLE 6, then blood samples were collected from the anesthetized rats. A midline incision was made on each animal, the colon was removed and length measured and recorded, a longitudinal incision was made on each colon, fecal material was gently washed away with saline, excess fluid was gently blotted and the colon weighed. The severity of IBD was assessed using the following scoring system: maximum score = 10

| 1) | Adhesions: |
|---|---|
| | none = 0 |
| | minimal = 1 |
| | involving several bowel loops = 2 |
| | |

| 2) | Strictures: |
|---|---|
| | none = 0 |
| | mild = 1 |
| | moderate = 2 |
| | severe, proximal dilatation = 3 |
| | |

| 3) | Ulcers: |
|---|---|
| | none = 0 |
| | inear ulceration < 1 cm = 1 |
| | two linear ulcers < 1 cm = 2 |
| | more sites of ulceration or one large ulcer = 3 |
| | |

| 4) | Wall thickness: |
|---|---|
| | less than 1 mm = 0 |
| | 1-3 mm = 1 |
| | > 3 mm = 2 |

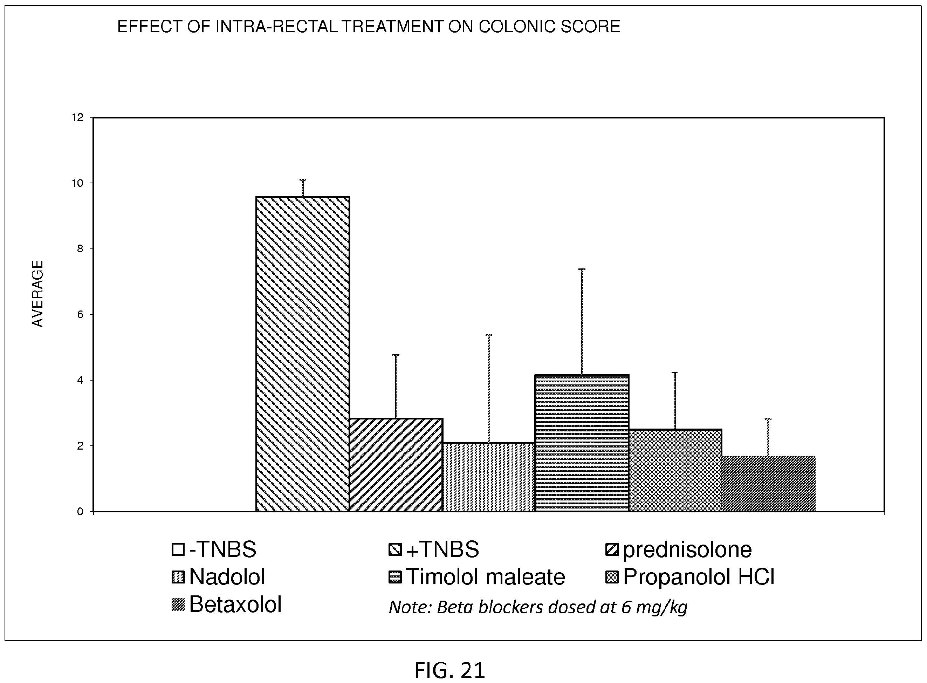

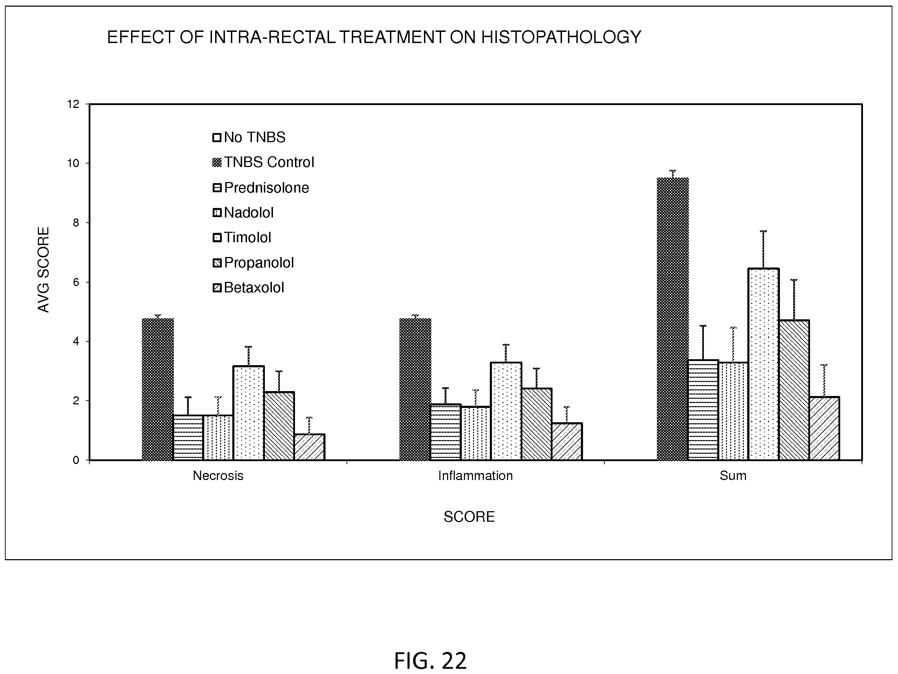

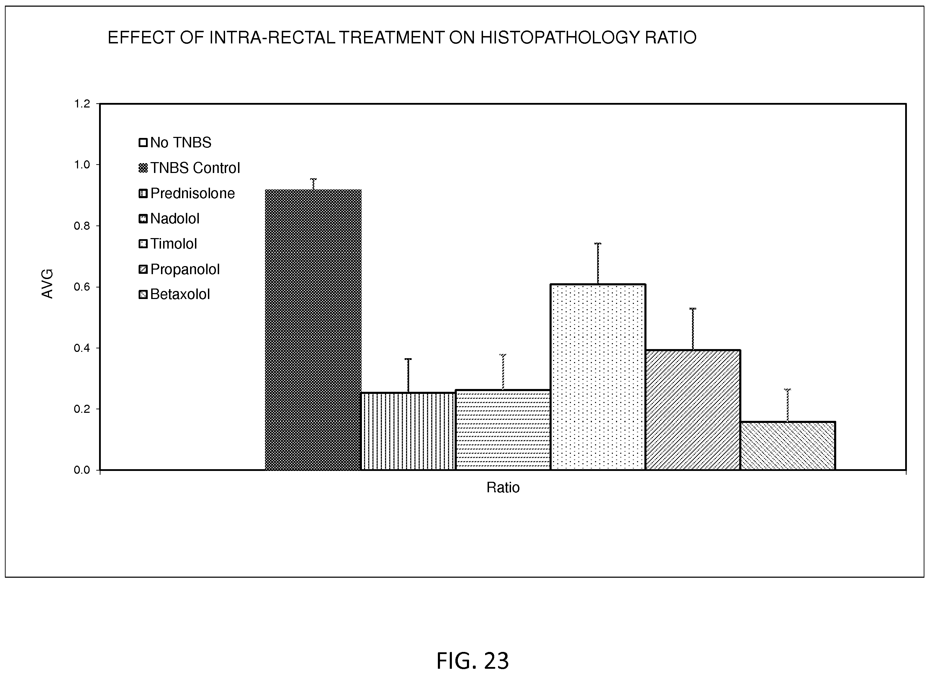

A section of colon was preserved in 10 volumes of 10% neutral buffered formalin for histopathology. A section of the colon is homogenized in PBS, centrifuged, and aliquots of the homogentat were frozen and stored for cytokine analysis. The results of this study were recorded and displayed in FIG. 21-23.

## Claims

1. Nadolol for use in treating an inflammatory bowel disorder in a human in need thereof, wherein the nadolol is formulated for release in the small intestine or the large intestine of the human.

2. Nadolol for use according to claim 1, wherein the inflammatory bowel disorder is colitis.

3. Nadolol for use according to claim 1 or 2, wherein the inflammatory bowel disorder is ulcerative colitis.

4. Nadolol for use according to any one of claims 1-3, wherein the nadolol is formulated for oral administration.

5. Nadolol for use according to any one of claims 1-3, wherein the nadolol is formulated for rectal administration.

6. Nadolol for use according to any one of claims 1-5, wherein the nadolol is formulated for administration in an amount of less than 40 mg daily.

7. Nadolol for use according to claim 6, wherein the nadolol is formulated for administration in an amount of less than 20 mg daily.

8. Nadolol for use according to any one of claims 1 - 7, wherein the nadolol is formulated for release in the small intestine.

9. Nadolol for use according to any one of claims 1 - 7, wherein the nadolol is formulated for release in the large intestine.

## Patentansprüche

1. Nadolol für die Verwendung bei der Behandlung einer chronisch-entzündlichen Darmerkrankung in einem Menschen, der diese benötigt, wobei das Nadolol für die Freisetzung im Dünndarm oder dem Dickdarm des Menschen formuliert ist.

2. Nadolol für die Verwendung nach Anspruch 1, wobei die chronisch-entzündliche Darmerkrankung eine Colitis ist.

3. Nadolol für die Verwendung nach Anspruch 1 oder 2, wobei die chronisch-entzündliche Darmerkrankung eine Colitis ulcerosa ist.

4. Nadolol für die Verwendung nach einem der Ansprüche 1-3, wobei das Nadolol für die orale Verabreichung formuliert ist.

5. Nadolol für die Verwendung nach einem der Ansprüche 1-3, wobei das Nadolol für die rektale Verabreichung formuliert ist.

6. Nadolol für die Verwendung nach einem der Ansprüche 1-5, wobei das Nadolol für die Verabreichung in einer Menge unter 40 mg pro Tag formuliert ist.

7. Nadolol für die Verwendung nach Anspruch 6, wobei das Nadolol für die Verabreichung in einer Menge unter 20 mg pro Tag formuliert ist.

8. Nadolol für die Verwendung nach einem der Ansprüche 1-7, wobei das Nadolol für die Freisetzung im Dünndarm formuliert ist.

9. Nadolol für die Verwendung nach einem der Ansprüche 1-7, wobei das Nadolol für die Freisetzung im Dickdarm formuliert ist.

## Revendications

1. Nadolol destiné à être utilisé dans le traitement d'un trouble inflammatoire intestinal chez un humain qui en a besoin, dans lequel le nadolol est formulé pour une libération dans l'intestin grêle ou le gros intestin de l'homme.

2. Nadolol destiné à être utilisé selon la revendication 1, dans lequel le trouble inflammatoire intestinal est la colite.

3. Nadolol destiné à être utilisé selon la revendication 1 ou 2, dans lequel le trouble inflammatoire intestinal est la colite ulcéreuse.

4. Nadolol destiné à être utilisé selon une quelconque des revendications 1 à 3, dans lequel le nadolol est formulé pour une administration orale.

5. Nadolol destiné à être utilisé selon une quelconque des revendications 1 à 3, dans lequel le nadolol est formulé pour une administration rectale.

6. Nadolol destiné à être utilisé selon une quelconque des revendications 1 à 5, dans lequel le nadolol est formulé pour une administration dans une quantité inférieure à 40 mg par jour.

7. Nadolol destiné à être utilisé selon la revendication 6, dans lequel le nadolol est formulé pour une administration dans une quantité inférieure à 20 mg par jour.

8. Nadolol destiné à être utilisé selon une quelconque des revendications 1 à 7, dans lequel le nadolol est formulé pour une libération dans l'intestin grêle.

9. Nadolol destiné à être utilisé selon une quelconque des revendications 1 à 7, dans lequel le nadolol est formulé pour une libération dans le gros intestin.
